(19) Europäisches Patentamt
European Patent Office
Office européen des brevets

(11) EP 1 746 540 A2

(12) EUROPEAN PATENT APPLICATION

(43) Date of publication:
24.01.2007 Bulletin 2007/04

(51) Int Cl.:
G06T 11/00 (2006.01)

(21) Application number: 06253679.2

(22) Date of filing: 13.07.2006

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK YU

(30) Priority: 20.07.2005 JP 2005210334

(71) Applicant: GE Medical Systems Global Technology Company, LLC
Waukesha,
Wisconsin 53188-1696 (US)

(72) Inventors:
• Nishide, Akihiko
Hino-shi, Tokyo 191-8503 (JP)
• Horiuchi, Tetsuya
Hino-shi, Tokyo 191-8503 (JP)
• Hagiwara, Akira
Hino-shi, Tokyo 191-8503 (JP)

(74) Representative: Goode, Ian Roy et al
London Patent Operation
General Electric International, Inc.
15 John Adam Street
London WC2N 6LU (GB)

(54) Image processing apparatus and X-ray CT apparatus

(57) The present invention is intended to improve the quality of a four-dimensional image that is a time-varying three-dimensional image. The four-dimensional image that is a time-varying three-dimensional image is spatially filtered in the direction of a time axis and the directions of spatial axes alike.

FIG. 3

**Description**

The present invention relates to an image processing apparatus that improves the quality of a time-varying image, or more particularly, to improvement in the quality of images produced through helical scanning or cine scanning performed by an X-ray CT apparatus, and reduction in a patient dose derived from the helical scanning or cine scanning.

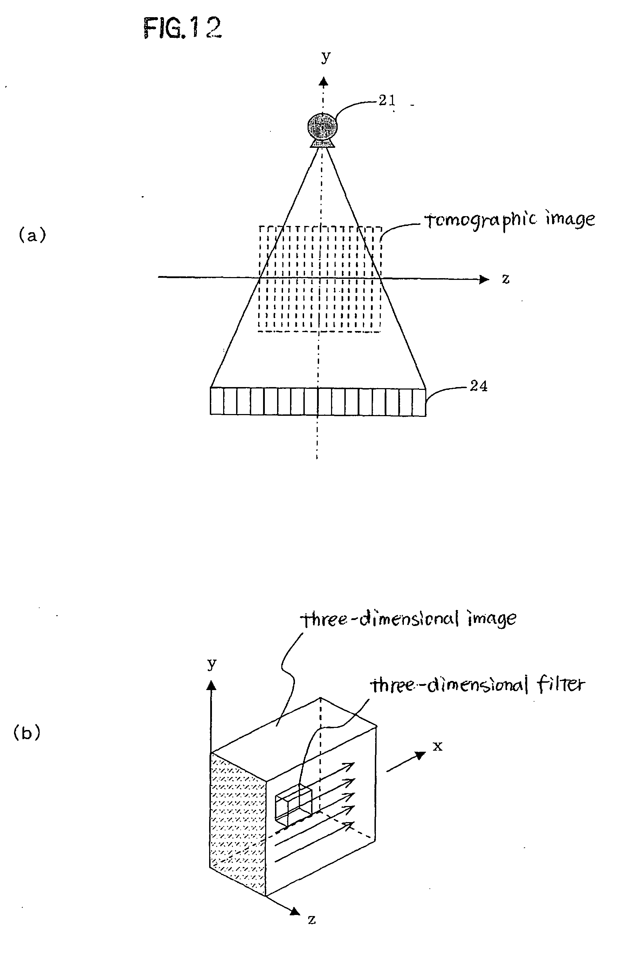

In an X-ray CT apparatus including a two-dimensional X-ray area detector represented by a multi-array X-ray detector or a flat-panel X-ray detector, a three-dimensional spatial filter defined in the directions of x, y, and z spatial axes as shown in Fig. 12(b) is applied to a three-dimensional image produced through cine scanning and defined in the directions of the x, y, and z axes as shown in Fig. 12(a). Noises are thus minimized. An example of the three-dimensional spatial filter is described in "Oplus E", Nov., 1988, pp.144-145, New Technological Communications Inc.

However, the related art is concerned with spatial filtering to be performed in the directions of three dimensions, that is, in the directions of x, y, and z axes but does not encompass time-sequential processing. Reduction in a patient X-ray dose and improvement in image quality have therefore been requested. From this viewpoint, the related art is not fully acceptable.

In general, improvement in image quality is constantly requested in the field of image processing.

Moreover, in the field of medical-purpose radiographic diagnosis, the issue of an unnecessary patient X-ray dose has become more and more controversial along with prevalence of X-ray CT apparatuses and diversity in purposes of examination. Reduction in a patient dose is requested all the time.

Therefore, an object of the present invention is to provide an image processing apparatus that uses pieces of information on the direction of a time axis and the directions of spatial axes to improve the quality of a four-dimensional image that is a time-varying three-dimensional image and that is composed of time-sequential three-dimensional images, a three-dimensional image that is a time-varying two-dimensional image and that is composed of time-sequential two-dimensional image, or an N-dimensional image that is a time-varying N-1-dimensional image and that is composed of time-sequential N-1-dimensional images.

Another object of the present invention is to provide an X-ray CT apparatus that includes a matrix-type two-dimensional area X-ray detector represented by a multi-array X-ray detector or a flat-panel X-ray detector, that offers target image quality with a smaller X-ray dose by improving the quality of time-sequential three-dimensional images or time-sequential two-dimensional images, which are produced through conventional (axial) scanning, cine scanning, or helical scanning, using pieces of information on the direction of a time axis and the directions of spatial axes.

The present invention provides an image processing method and an image processing apparatus that can improve the quality of a four-dimensional image that is a time-varying three-dimensional image, a three-dimensional image that is a time-varying two-dimensional image, or an N-dimensional image that is a time-varying N-1-dimensional image (an N-1-dimensional image defined with N-1 independent parameters as a base) by performing spatial filtering or adaptive spatial filtering in the direction of a time axis and the directions of spatial axes.

According to the present invention, time-sequential three-dimensional images or time-sequential two-dimensional images produced through cine scanning or helical scanning performed by an X-ray CT apparatus including a matrix-type two-dimensional area X-ray detector represented by a multi-array X-ray detector or a flat-panel X-ray detector are spatially filtered in the direction of a time axis and the directions of spatial axes. Otherwise, adaptive spatial filtering that filters only pixels belonging to a homogeneous domain is performed in order to improve image quality.

According to the first aspect of the present invention, there is provided an image processing apparatus including an image input means for receiving a time-varying three-dimensional image, a spatial filter means for performing four-dimensional spatial filtering in the direction of a time axis and the directions of spatial axes, and an image output/display means for transmitting or displaying a spatially filtered three-dimensional image.

The image processing apparatus in accordance with the first aspect uses a four-dimensional spatial filter to treat pixels mutually neighboring not only in the directions of spatial axes that are x, y, and z axes but also in the direction of a time axis. For reduction of noises in an image, the noises can be more effectively reduced by employing many pixels.

According to the second aspect of the present invention, there is provided an image processing apparatus including an image input means for receiving a time-varying two-dimensional image, a spatial filter means for performing three-dimensional spatial filtering in the direction of a time axis and the directions of spatial axes, and an image output/display means for transmitting or displaying a spatially filtered two-dimensional image.

The image processing apparatus in accordance with the second aspect uses a three-dimensional spatial filter to treat pixels mutually neighboring not only in the directions of spatial axes that are x and y axes but also in the direction of a time axis. For reduction of noises in an image, the noises can be more effectively reduced by employing many pixels.

According to the third aspect of the present invention, there is provided an image processing apparatus including an image input means that receives a time-varying N-1-dimensional image which is defined with N-1 time-varying independent parameters as a base, a spatial filter means for performing N-dimensional spatial filtering in the direction of a time axis and the directions of spatial axes, and an image output/display means for transmitting or displaying a spatially filtered N-1-dimensional image.

The image processing apparatus in accordance with the third aspect uses an N-dimensional spatial filter to treat pixels mutually neighboring in the directions of axes in an N-1-dimensional space and the direction of a time axis. For reduction of noises in an image, the noises can be more effectively reduced by employing many pixels.

According to the fourth aspect of the present invention, there is provided an image processing apparatus including an image input means for receiving a time-varying three-dimensional image, a spatial filter means for selecting pixels that mutually neighbor in the direction of a time axis and the directions of spatial axes, and performing adaptive four-dimensional filtering on the selected neighboring pixels, and an image output/display means for transmitting or displaying a spatially filtered three-dimensional image.

The image processing apparatus in accordance with the fourth aspect uses a four-dimensional spatial filter to treat pixels mutually neighboring not only in the directions of spatial axes that are x, y, and z axes but also in the direction of a time axis. For reduction of noises in an image, the noises can be more effectively reduced by selecting intended pixels from among many pixels.

According to the fifth aspect of the present invention, there is provided an image processing apparatus including an image input means for receiving a time-varying two-dimensional image, a spatial filter means for selecting pixels mutually neighboring in the direction of a time axis and the directions of spatial axes, and performing adaptive three-dimensional spatial filtering on the selected neighboring pixels, and an image output/display means for transmitting or displaying a spatially filtered two-dimensional image.

The image processing apparatus in accordance with the fifth aspect uses a three-dimensional spatial filter to treat pixels mutually neighboring not only in the directions of spatial axes that are x and y axes but also in the direction of a time axis. For reduction of noises in an image, the noise can be more effectively reduced by selecting intended pixels from among many pixels.

According to the sixth aspect of the present invention, there is provided an image processing apparatus including an image input means for receiving a time-varying N-1-dimensional image that is defined with N-1 time-varying independent parameters as a base, a spatial filter means for selecting pixels that mutually neighbor in the direction of a time axis and the directions of spatial axes, and performing adaptive N-dimensional spatial filtering on the selected neighboring pixels, and an image output/display means for transmitting or displaying a spatially filtered N-1-dimensional image.

The image processing apparatus in accordance with the sixth aspect uses an N-dimensional spatial filter to treat pixels mutually neighboring not only in the directions of axes in an N-1-dimensional space but also in the direction of a time axis. For reduction of noises in an image, the noises can be more effectively reduced by selecting intended pixels from among many pixels.

According to the seventh aspect of the present invention, there is provided an image processing apparatus identical to the image processing apparatus in accordance with any of the first to sixth aspects except that it comprises the spatial filter means which selects pixels whose values are statistically close to the value of a focused pixel aligned with the center of a spatial filter as the selected neighboring pixels,.

The image processing apparatus in accordance with the seventh aspect can more effectively reduce noises because when pixels mutually neighboring in the directions of spatial axes and the direction of a time axis are treated, homogeneous pixels are sampled and treated.

According to the eighth aspect of the present invention, there is provided an X-ray CT apparatus including: a data acquisition means that has an X-ray generator and a two-dimensional X-ray area detector, which is opposed to the X-ray generator and has a matrix structure, rotated about a center of rotation located between the X-ray generator and X-ray area detector so as to acquire projection data items of a subject lying down between the X-ray generator and X-ray area detector; an image reconstruction means for reconstructing an image according to acquired projection data items; a post-processing means for performing post-processing on a reconstructed tomographic image; a tomographic image display means for displaying a tomographic image having undergone post-processing; and a radiographic condition designation means for designating radiographic conditions. The post-processing means spatially filters a time-varying three-dimensional image, which is produced through tomography, in the direction of a time axis and the directions of spatial axes, that is, in x, y, and z directions where the z direction is a direction perpendicular to an xy plane that is a plane on which a data acquisition system rotates or a plane represented by a tomographic image.

The X-ray CT apparatus in accordance with the eighth aspect performs four-dimensional spatial filtering or three-dimensional spatial filtering on time-sequential three-dimensional images or time-sequential two-dimensional images, which are produced through tomography, in the direction of a time axis and the directions of spatial axes in an image space so as to improve image quality and reduce a patient dose. Otherwise, the four-dimensional spatial filtering or three-dimensional spatial filtering is performed on only homogeneous pixels neighboring a focused pixel.

According to the ninth aspect of the present invention, there is provided an X-ray CT apparatus including: a data acquisition means that has an X-ray generator and a two-dimensional X-ray area detector, which is opposed to the X-ray generator and has a matrix structure, about a center of rotation located between the X-ray generator and X-ray area detector so as to acquire projection data items of a subject lying down between the X-ray generator and X-ray area detector; an image reconstruction means for reconstructing an image according to acquired projection data items; a post-processing means for performing post-processing on a reconstructed tomographic image; a tomographic image display means for displaying a tomographic image having undergone post-processing; and a radiographic condition designation means for designating radiographic conditions. The X-ray CT apparatus further includes a preprocessing means that performs spatial filtering on time-varying projection data items, which are produced through tomography, in the direction of a time axis and the directions of spatial axes, that is, the direction of channels, the direction of detector arrays, and a direction determined by a view angle.

The X-ray CT apparatus in accordance with the ninth aspect performs four-dimensional spatial filtering or three-dimensional spatial filtering in the direction of a time axis and the directions of spatial axes in a space, in which time-sequential three-dimensional projection data items or time-sequential two-dimensional projection data items which are produced through tomography are defined, so as to improve image quality and reduce a patient dose. Otherwise, the four-dimensional spatial filtering or three-dimensional spatial filtering is performed only on homogeneous pixels neighboring a focused pixel.

According to the tenth aspect of the present invention, there is provided an X-ray CT apparatus including: a data acquisition means that has an X-ray generator and a two-dimensional X-ray area detector, which is opposed to the X-ray generator and has a matrix structure, about a center of rotation located between the X-ray generator and X-ray area detector so as to acquire projection data items of a subject lying down between the X-ray generator and X-ray area detector; an image reconstruction means for reconstructing an image according to acquired projection data items; a post-processing means for performing post-processing on a reconstructed tomographic image; a tomographic image display means for displaying a tomographic image having undergone post-processing; and a radiographic condition designation means for designating radiographic conditions. The post processing means includes a means for selecting pixels, which mutually neighbor in the direction of a time axis and the directions of spatial axes, that is, in x, y, and z directions where the z direction is a direction perpendicular to an xy plane that is a plane on which a data acquisition system rotates or a plane represented by a tomographic image, from among pixels contained in time-varying three-dimensional image data that is produced through tomography, and a means for performing adaptive spatial filtering on the selected neighboring pixels.

The X-ray CT apparatus in accordance with the tenth aspect performs four-dimensional or three-dimensional adaptive spatial filtering on time-sequential two-dimensional images or time-sequential three-dimensional images, which are produced through tomography, in the direction of a time axis and the directions of spatial axes in an image space so as to improve image quality and reduce a patient dose.

According to the eleventh aspect of the present invention, there is provided an X-ray CT apparatus including: a data acquisition means that has an X-ray generator and a two-dimensional X-ray area detector, which is opposed to the X-ray generator and has a matrix structure, about a center of rotation located between the X-ray generator and X-ray area detector so as to acquire projection data items of a subject lying down between the X-ray generator and X-ray area detector; an image reconstruction means for reconstructing an image according to acquired projection data items; a post-processing means for performing post-processing on a reconstructed tomographic image; a tomographic image display means for displaying a tomographic image having undergone post-processing; and a radiographic condition designation means for designating radiographic conditions. The X-ray CT apparatus further includes a preprocessing means composed of a means for selecting pixels, which mutually neighbor in the direction of a time axis and the directions of spatial axes, that is, in x, y, and z directions where the z direction is a direction perpendicular to an xy plane that is a plane on which a data acquisition system rotates or a plane represented by a tomographic image, from among pixels contained in time-varying projection data items that are produced through tomography, and a means for performing adaptive spatial filtering on the selected neighboring pixels.

The X-ray CT apparatus in accordance with the eleventh aspect performs four-dimensional or three-dimensional adaptive spatial filtering on time-sequential two-dimensional images or time-sequential three-dimensional images, which are produced through tomography, in the direction of a time axis and the directions of spatial axes in a projection data space so as to improve image quality and reduce a patient dose.

According to the twelfth aspect of the present invention, there is provided an X-ray CT apparatus identical to the X-ray CT apparatus according to any of the eighth to eleventh aspects except that as the selected neighboring pixels, pixels whose values are statistically close to the value of a focused pixel aligned with the center of a spatial filter are selected.

The X-ray CT apparatus in accordance with the twelfth aspect more effectively reduces noises because when pixels mutually neighboring not only in the directions of spatial axes but also in the direction of a time axis are treated, homogeneous pixels are sampled and then treated.

According to the thirteenth aspect of the present invention, there is provided an X-ray CT apparatus identical to the X-ray CT apparatus according to any of the eighth to twelfth aspects except that it comprises the data acquisition means which includes as the two-dimensional X-ray area detector having a matrix structure an arc-shaped multi-array X-ray detector.

Owing to the arc-shaped multi-array X-ray detector, the X-ray CT apparatus in accordance with the thirteenth aspect can produce a plurality of tomographic images, which expresses sections of a subject mutually succeeding in a z direction, during one rotational data acquisition so as to reconstruct a three-dimensional image. A plurality of rotational data acquisitions provides time-sequential three-dimensional images.

According to the fourteenth aspect of the present invention, there is provided an X-ray CT apparatus identical to the X-ray CT apparatus according to any of the eighth to twelfth aspects except that it comprises the data acquisition means which includes as the two-dimensional X-ray area detector having a matrix structure one planar two-dimensional X-ray area detector or a plurality of planar two-dimensional X-ray area detectors.

Owing to the two-dimensional X-ray area detector realized with one planar two-dimensional X-ray area detector or a plurality of planar two-dimensional X-ray area detectors, the X-ray CT apparatus in accordance with the fourteenth aspect can produce a plurality of tomographic images, which expresses sections of a subject mutually succeeding in a z direction, during one rotational data acquisition, and thus reconstruct a three-dimensional image. Moreover, a plurality of rotational data acquisitions provides time-sequential three-dimensional images.

According to the fifteenth aspect of the present invention, there is provided an X-ray CT apparatus identical to the X-ray CT apparatus according to any of the eighth to fourteenth aspects except that it comprises the image reconstruction means which adopts three-dimensional image reconstruction as the image reconstruction.

Since the X-ray CT apparatus in accordance with the fifteenth aspect adopts three-dimensional image reconstruction as image reconstruction, even when a two-dimensional X-ray area detector that is wide in a z direction is employed, a tomographic image that is more homogeneous in the z direction can be reconstructed. Time-sequential three-dimensional images produced based on tomographic images are therefore homogeneous in the z direction. Four-dimensional or three-dimensional spatial filtering can therefore be more effectively performed in the direction of a time axis and the directions of spatial axes. Moreover, when three-dimensional image reconstruction is adopted, a plurality of tomographic images expressing sections of a subject falling within a wide range in the z direction can be reconstructed through helical scanning. In general, when a point on the time axis changes to another point during the helical scanning, the ranges of sections in the z direction expressed by three-dimensional images overlap to a limited extent. In contrast, when a point on the time axis changes to another point during the helical scanning, the ranges of sections in the z direction expressed by three-dimensional images overlap to a large extent. Eventually, four-dimensional or three-dimensional spatial filtering can be more effectively performed in the direction of the time axis and the directions of spatial axes alike.

According to the sixteenth aspect of the present invention, there is provided an X-ray CT apparatus identical to the X-ray CT apparatus according to any of the eighth to fifteenth aspects except that it comprises the post-processing means which performs post-processing on a tomographic image produced through cine scanning.

When the X-ray CT apparatus in accordance with the sixteenth aspect performs cine scanning using the two-dimensional X-ray area detector, a plurality of sets of tomographic images each expressing a range of sections of a subject that has a certain width in the z direction is reconstructed time-sequentially. The plurality of sets of tomographic images constitutes time-sequential three-dimensional images. Four-dimensional or three-dimensional spatial filtering can be performed in the direction of a time axis and the directions of spatial axes alike.

According to the seventeenth aspect of the present invention, there is provided an X-ray CT apparatus identical to the X-ray CT apparatus according to the eighth to fifteenth aspects except that it comprises the post-processing means which performs post-processing on a tomographic image produced through helical scanning.

The X-ray CT apparatus in accordance with the seventeenth aspect includes the two-dimensional X-ray area detector. When three-dimensional image reconstruction is adopted as image reconstruction, a plurality of tomographic images expressing a range of sections of a subject, which has a certain width in the z direction, is reconstructed at a certain time instant through helical scanning. Especially when a helical pitch is set to 1 or less, a range having a certain width in the z direction at a time instant overlaps a range having a certain width in the z direction at the next time instant to a great extent. Four-dimensional or three-dimensional spatial filtering can be performed on images, which express sections falling within a duplicate portion shared by the overlapping ranges, in the direction of a time axis and the directions of spatial axes alike.

According to the eighteenth aspect of the present invention, there is provided an X-ray CT apparatus identical to the X-ray CT apparatus according to any of the eighth to seventeenth aspects except that it comprises: the radiographic condition designation means which receives a noise index value; and the post-processing means which optimizes time-sequential three-dimensional (four-dimensional) spatial filtering on the basis of the noise index value for the purpose of post-processing.

In the X-ray CT apparatus according to the eighteenth aspect, when a subject whose shape varies region by region in the z direction is scanned, even if radiographic conditions are held intact, image quality affected by noises in an image is not constant among images of sections juxtaposed in the z direction. Consequently, parameters that define four-dimensional or three-dimensional spatial filtering and that are concerned with the direction of a time axis and the directions of spatial axes are varied with a noise index value, which is designated as one of radiographic conditions, as a target value. Thus, the four-dimensional or three-dimensional spatial filtering is optimized for each position in the z direction. Eventually, the image quality becomes nearly uniform in the z direction.

According to the nineteenth aspect of the present invention, there is provided an X-ray CT apparatus identical to the X-ray CT apparatus according to any of the eighth to eighteenth aspects except that it comprises the radiographic condition designation means which optimizes radiographic conditions according to a noise index value.

In the X-ray CT apparatus according to the nineteenth aspect, when a subject whose shape varies region by region in the z direction is scanned, if image quality must be held constant in the z direction, a noise index value designated as one of radiographic conditions is used as a target value to optimize parameters that define four-dimensional or three-dimensional spatial filtering and that are concerned with the direction of a time axis and the directions of spatial axes. If the image quality does not become nearly uniform in the z direction, the radiographic conditions are varied in the z direction in order to make the image quality uniform in the z direction. When a tube current is varied in the z direction and spatial filtering is performed, the image quality becomes nearly uniform in the z direction.

As an advantage provided by the present invention, pieces of information on the direction of a time axis and the directions of all spatial axes can be used to improve the quality of a four-dimensional image that is a time-varying three-dimensional image and is composed of time-sequential three-dimensional images, a three-dimensional image that is a time-varying two-dimensional image and is composed of time-sequential two-dimensional images, or an N-dimensional image that is a time-varying N-1-dimensional image and is composed of time-sequential N-1-dimensional images.

Moreover, as another advantage provided by the present invention, pieces of information on the direction of a time axis and the directions of spatial axes can be used to improve the quality of time-sequential three-dimensional images or time-sequential two-dimensional images that are produced through conventional (axial) scanning or cine scanning performed by an X-ray CT apparatus including a matrix-type two-dimensional area X-ray detector represented by a multi-array X-ray detector or a flat-panel X-ray detector. Consequently, target image quality can be realized with a smaller X-ray dose.

Further objects and advantages of the present invention will be apparent from the following description of the preferred embodiments of the invention as illustrated in the accompanying drawings, in which:

Fig. 1 is a block diagram showing an X-ray CT apparatus in accordance with an embodiment of the present invention.

Fig. 2 is an explanatory diagram showing the rotation of an X-ray generator (X-ray tube) and a multi-array X-ray detector.

Fig. 3 is a flowchart outlining actions to be performed in the X-ray CT apparatus in accordance with the embodiment of the present invention.

Fig. 4 is a flowchart describing preprocessing.

Fig. 5 is a flowchart describing three-dimensional image reconstruction.

Fig. 6 includes conceptual diagrams showing projection of lines in a field of view in a direction of X-ray transmission.

Fig. 7 is a conceptual diagram showing lines projected on the surface of a detector.

Fig. 8 is a conceptual diagram showing projection of projection data items Dr(view,x,y) on the field of view.

Fig. 9 is a conceptual diagram showing back projection pixel data items D2 representing the pixel points in the field of view.

Fig. 10 is an explanatory diagram showing production of back projection data items D3 by summating sets of back projection pixel data items D2, which are produced from all views, pixel by pixel.

Fig. 11 includes conceptual diagrams showing projection of lines in a circular field of view in the direction of X-ray transmission.

Fig. 12 shows a conventional three-dimensional image filter.

Fig. 13(a) shows tomographic images produced at respective time instants through helical scanning, and Fig. 13(b) shows tomographic images produced at respective time instants through cine scanning.

Fig. 14(a) shows a three-dimensional image in which a slice thickness of tomographic images corresponds to an inter-tomographic image spacing, and

Fig. 14(b) shows a three-dimensional image in which the slice thickness is larger than the inter-tomographic image spacing.

Fig. 15 is an explanatory diagram concerning four-dimensional sweeping of a four-dimensional spatial filter.

Fig. 16 shows a neighborhood (eighty neighbors) to which a four-dimensional spatial filter is applied.

Fig. 17 shows a neighborhood (six hundreds and twenty-four neighbors) to which the four-dimensional spatial filter is applied.

Fig. 18 shows an example of a four-dimensional spatial filter (having three coefficients defined in four dimensions) intended to reduce noises.

Fig. 19 shows an example of the four-dimensional spatial filter (having five coefficients defined in four dimensions) intended to reduce noises.

Fig. 20 shows an example of a four-dimensional spatial filter intended to reduce noises and dependent on a CT number.

Fig. 21 shows an example of a four-dimensional spatial filter intended to enhance a contrast and reduce noises and dependent on a CT number.

Fig. 22 is a flowchart describing spatial filtering dependent on a pixel value (CT number).

Fig. 23 shows a flowchart describing spatial filtering dependent on the property of a neighborhood.

Fig. 24 shows an example of three-dimensional MPR display or three-dimensional display.

Fig. 25(a) shows a case where the property of a neighborhood resembles that of a focused pixel, Fig. 25(b) shows a case where the property of the neighborhood does not resemble that of the focused pixel, and Fig. 25(c) shows the focused pixel and neighborhood.

The present invention will be described by taking an illustrated embodiment for instance. Noted is that the present invention will not be limited to the embodiment.

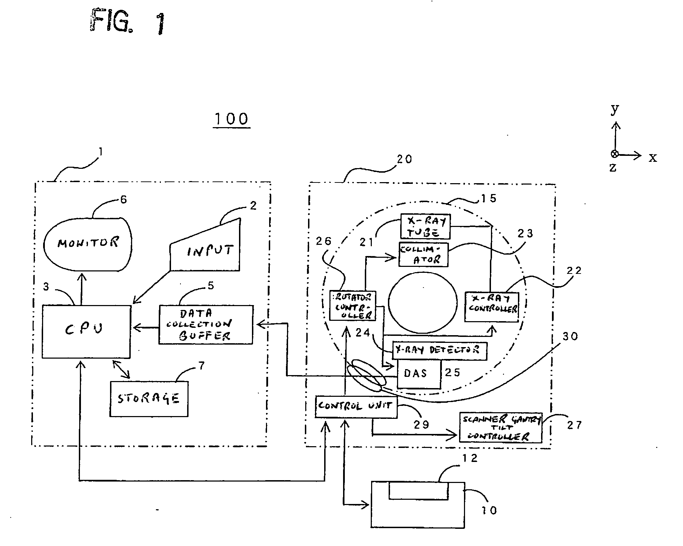

Fig. 1 is a block diagram showing the configuration of an X-ray CT apparatus in accordance with an embodiment of the present invention. The X-ray CT apparatus 100 includes an operator console 1, a radiographic table 10, and a scanner gantry 20.
The operator console 1 includes an input device 2 that receives an operator's entry, a central processing unit 3 that performs preprocessing, image reconstruction, post-processing, and others, a data collection buffer 5 in which X-ray detector data items acquired by the scanner gantry 20 are collected, a monitor 6 on which a reconstructed tomographic image is displayed according to projection data items produced by performing preprocessing on the X-ray detector data items, and a storage device 7 in which programs, X-ray detector data items, projection data items, and X-ray tomographic images are stored.
The radiographic table 10 has a cradle 12 on which a subject lies down and which is inserted into or drawn out of the bore of the scanner gantry 20. The cradle 12 is lifted, lowered, or moved rectilinearly with respect to the radiographic table 10 by a motor incorporated in the radiographic table 10.
The scanner gantry 20 includes an X-ray tube 21, an X-ray controller 22, a collimator 23, a multi-array X-ray detector 24, a data acquisition system (DAS) 25, a rotator controller 26 that controls the X-ray tube 21 and others which rotate about the body axis of a subject, and a control unit 29 that transfers control signals to or from the operator console 1 and radiographic table 10. A scanner gantry tilt controller 27 allows the scanner gantry 20 to tilt forward or backward at approximately ±30° with respect to the z direction.

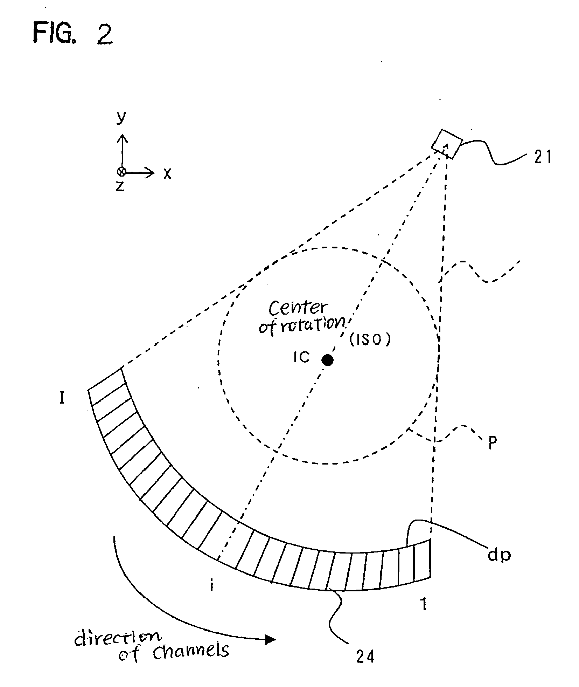

Fig. 2 is an explanatory diagram showing the geometric arrangement of the X-ray tube 21 and multi-array X-ray detector 24.
The X-ray tube 21 and multi-array X-ray detector 24 rotate about a center of rotation IC. Assuming that a vertical direction is a y direction, a horizontal direction is an x direction, and a table-advancing direction perpendicular to the x and y directions is a z direction, a plane on which the X-ray tube 21 and multi-array X-ray detector 24 rotate is an xy plane. Moreover, a moving direction in which the cradle 12 moves is the z direction.

The X-ray tube 21 generates an X-ray beam that is called a cone beam CB. When the direction of the center axis of the cone beam CB is parallel to the y direction, the X-ray tube shall be located at a view angle of 0°.

The multi-array X-ray detector 24 includes, for example, 256 detector arrays. Each detector array has, for example, 1024 detector channels. In other words, the multi-array X-ray detector 24 has a plurality of X-ray detector elements, which detect X-rays, arrayed in the form of a matrix or juxtaposed in both the direction of channels in which the X-ray tube 21 is moved by the rotator 15 so that the X-ray tube will rotate about a subject and the direction of arrays corresponding to the direction of the axis of the rotation of the X-ray tube 21 made about a subject by the rotator 15.

X-rays are irradiated and projection data items are produced by the multi-array X-ray detector 24. The projection data items are then analog-to-digital converted by the DAS 25, and transferred to the data collection buffer 5 via a slip ring 30. The data items transferred to the data collection buffer 5 are treated by the central processing unit 3 according to a program read from the storage device 7. A tomographic image is then reconstructed and displayed on the monitor 6.

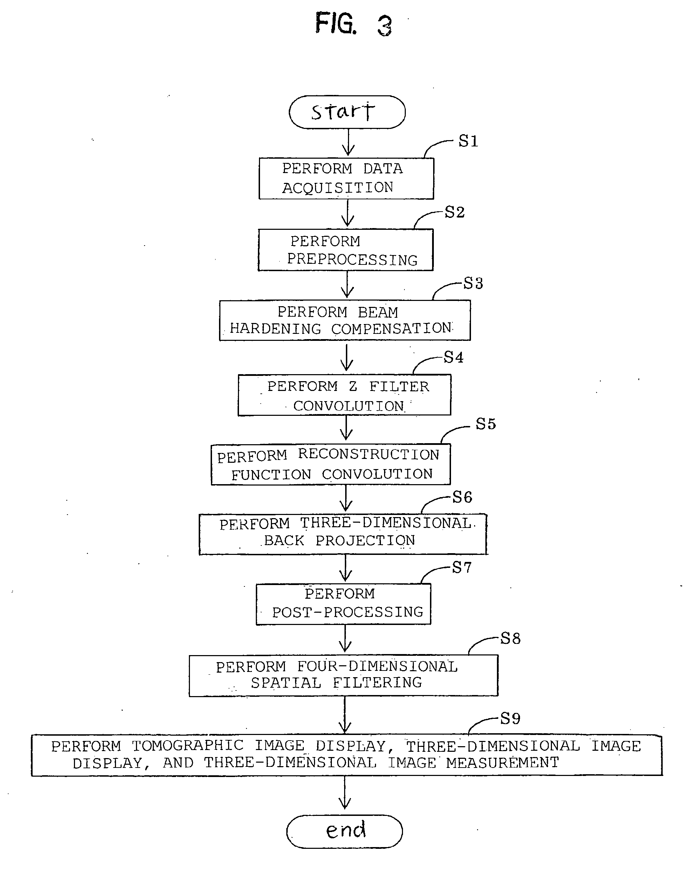

Fig. 3 is a flowchart outlining actions to be performed in the X-ray CT apparatus 100 in accordance with the present embodiment.

At step S1, assuming that helical scanning is adopted, the X-ray tube 21 and multi-array X-ray detector 24 are rotated about a subject. While the cradle 12 is rectilinearly moved on the radiographic table 10, X-ray detector data items are acquired. At this time, a table position in the z direction of rectilinear movement Ztable(view) is appended to each of X-ray detector data items D0(view,j,i) which is identified with a view angle view, a detector array number j, and a channel number i. When conventional (axial) scanning or cine scanning is adopted, the cradle 12 on the radiographic table 10 is immobilized at a certain position in the z direction. A data acquisition system is rotated once or a plurality of times in order to acquire X-ray detector data items. If necessary, after the cradle is moved to the next position in the z direction, the data acquisition system is rotated once or a plurality of times in order to acquire X-ray detector data items. Incidentally, the view angle view is an angle by which the X-ray tube 21 is rotated about the subject from a predetermined position by the rotator 15. Moreover, the detector array number j is a number assigned to each X-ray detector array of detector elements that are included in the multi-array X-ray detector 24 and that are juxtaposed in the direction of arrays. The channel number i is a number assigned to detector elements that are included in the multi-array X-ray detector 24 and that are juxtaposed in the direction of channels. Moreover, the X-ray detector data D0(view,j,i) is data of X-rays detected by a detector element or on a channel, which belongs to an X-ray detector array j and a channel i in the multi-array X-ray detector 24, when the X-ray tube 21 located at a predetermined view angle view irradiates X-rays to the subject. The table position in the z direction of rectilinear movement Ztable(view) is a position to which the cradle 12 of the radiographic table 10 is moved in the direction z of the subject's body axis during a scan.

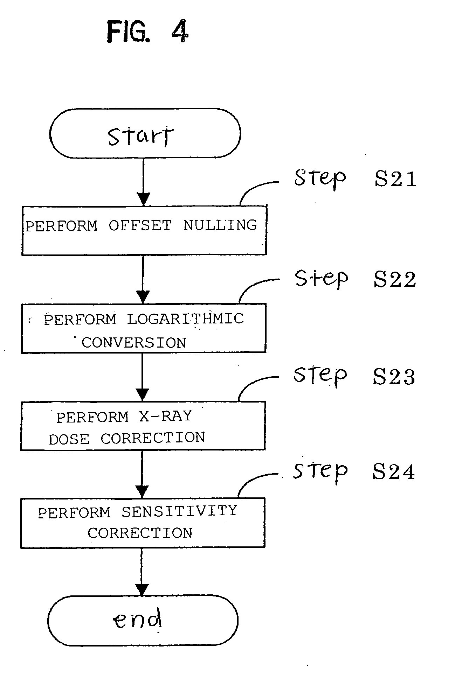

At step S2, the X-ray detector data items D0(view,j,i) are preprocessed and converted into projection data items. The preprocessing includes, as described in Fig. 4, offset nulling of step S21, logarithmic conversion of step S22, X-ray dose correction of step S23, and sensitivity correction of step S24.

At step S3, an effect of beam hardening on the preprocessed projection data items D1(view,j,i) is compensated. Assuming that D1(view,j,i) denotes projection data items having undergone the sensitivity correction S24 included in the preprocessing S2 and D11(view,j,i) denotes data items having undergone the beam hardening compensation S3, the beam hardening compensation of step S3 is expressed by the formula (1) that is a polynomial expression.

$$D11(view,j,i) =$$

$$D1(view,j,i) \cdot (B_0(j,i) + B_1(j,i) \cdot D_1(view,j,i) + B_2(j,i) \cdot$$

$$D1(view,j,i)^2 \qquad\qquad (1)$$

At this time, since the beam hardening compensation is performed on each detector array j included in the X-ray detector, if a tube voltage that is one of radiographic conditions is differentiated from data acquisition to data acquisition, a difference in an X-ray energy characteristic of one detector array from another can be compensated.

At step S4, z filter convolution is performed in order to filter projection data items D11(view,j,i), which have undergone the beam hardening compensation, in the z direction (direction of arrays).

At step S4, a filter whose size in the direction of arrays corresponds to five arrays, for example, a filter having coefficients w1(ch), w2(ch), w3(ch), w4(ch), and w5(ch) defined in the direction of arrays is applied to projection data items D11(view,j,i) (where i ranges from 1 to CH and j ranges from 1 to ROW) that have been produced by preprocessing data items acquired by the multi-array X-ray detector during each data acquisition with the X-ray tube set at each view angle, and that have undergone the beam hardening compensation. The filter is expressed by the formula (2). Herein, i denotes a channel number and j denotes an array number.

$$\sum_{k=1}^{5} w_k(j) = 1 \qquad (2)$$

The corrected data items D12(view,j,i) are expressed by the formula (3).

$$D12(view,j,i) = \sum_{k=1}^{5} (D11(view, j-k-3, i) \bullet W_k(j)) = \qquad (3)$$

Assuming that the maximum channel number is CH and the maximum array number is ROW, the formulae (4) and (5) presented below are drawn out.

$$D11(view,-1,i) = D11(view,0,i) = D11(view,1,i) \qquad (4)$$

$$D11(view,ROW,i)=D11(view,ROW+1,i)=D11(view,ROW+2,i)$$

$$(5)$$

Moreover, a slice thickness can be controlled based on a distance from a center of image reconstruction by changing the filtering coefficients, which are applied in the direction of arrays, channel by channel. In general, the slice thickness of a tomographic image is larger in the perimeter thereof than in the center of reconstruction thereof. The filtering coefficients to be applied in the direction of arrays are differentiated between the center of a tomographic image and the perimeter thereof. The filtering coefficients to be applied to data items acquired by the detector elements located on and near the center channel in the direction of arrays are determined to have a large variance, while the filtering coefficients to be applied to data items acquired by the detector elements located on and near a perimetric channel in the direction of arrays are determined to have a small variance. Thus, the slice thickness of the perimeter of a tomographic image and that of the center of reconstruction thereof become close to each other.

When the filtering coefficients to be applied in the direction of arrays are, as mentioned above, controlled so that they will be different between data items acquired on and near the center channel of the multi-array X-ray detector 24 and those acquired on and near the perimetric channel thereof, the difference in a slice thickness between the center of a tomographic image and the perimeter thereof can be controlled. When the filtering coefficients to be applied in the direction of arrays are controlled in order to slightly increase the slice thickness, both artifacts and noises are largely reduced. Consequently, a degree to which artifacts or noises are reduced can be controlled. Namely, the quality of tomographic images to be reconstructed as a three-dimensional image, that is, the image quality attained on the xy plane can be controlled. As another embodiment, the filtering coefficients to be applied in the direction of arrays (z direction) may be determined to realize a de-convolution filter in order to produce a tomographic image of a small slice thickness.

At step S5, reconstruction function convolution is performed. Specifically, data items are Fourier-transformed, applied a reconstruction function, and then inverse-Fourier-transformed. Assuming that D12(view,j,i) denotes data items having undergone z filter convolution, D13(view,j,i) denotes data items having undergone reconstruction function convolution, and Kernel(j) denotes the reconstruction function, the reconstruction function convolution S5 is expressed by the formula (6).

$$D13(view,j,i) = D12(view,j,i) * Kernel(j) \qquad (6)$$

Since the reconstruction function Kernel(j) can be independently convoluted to data items acquired by each detector array j, a difference of the characteristic of one detector array concerning noises and resolution from that of another detector array can be compensated.

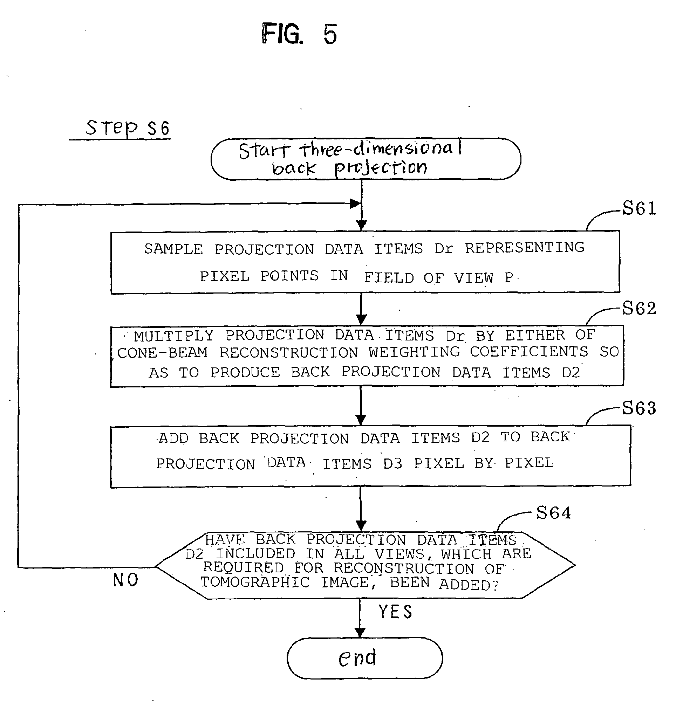

At step S6, three-dimensional back projection is performed on projection data items D13(view,j,i) having undergone reconstruction function convolution in order to produce back projection data items D3(x,y). A reconstructed image is a three-dimensional image representing a portion of a subject parallel to a plane perpendicular to the z axis, that is, the xy plane. Hereinafter, a field of view P shall be parallel to the xy plane. The three-dimensional back projection will be described later with reference to Fig. 5.

At step S7, post-processing including image filter convolution and CT number transform is performed on the back projection data items D3(x,y,z) in order to produce tomographic image data D31 (x,y).

Assuming that D31 (x,y,z) denotes tomographic image data having undergone three-dimensional back projection, D32(x,y,z) denotes data items having undergone image filter convolution, and Filter(z) denotes an image filter, the image filter convolution included in the post-processing is expressed by the formula (7).

$$D32(x,y,z) = D31(x,y,z) * Filter(z) \qquad (7)$$

Since the image filter can be independently convoluted to data items produced by each detector array, a difference of the characteristic of each detector array concerning noises and a resolution from that of another detector array can be compensated.

A tomographic image is displayed on the monitor 6 according to the resultant tomographic image data.

Fig. 5 is a flowchart describing three-dimensional back projection (step S6 in Fig. 4).

In the present embodiment, a reconstructed image is a three-dimensional image representing a portion of a subject parallel to a plane perpendicular to the z axis, that is, the xy plane. A field of view P shall be parallel to the xy plane.

At step S61, one of all views needed to reconstruct a tomographic image (that is, views produced with the X-ray tube rotated 360° or 180° + the angle of a fan-shaped beam) is focused, and projection data items Dr representing pixel points in the field of view P are sampled from the focused view.

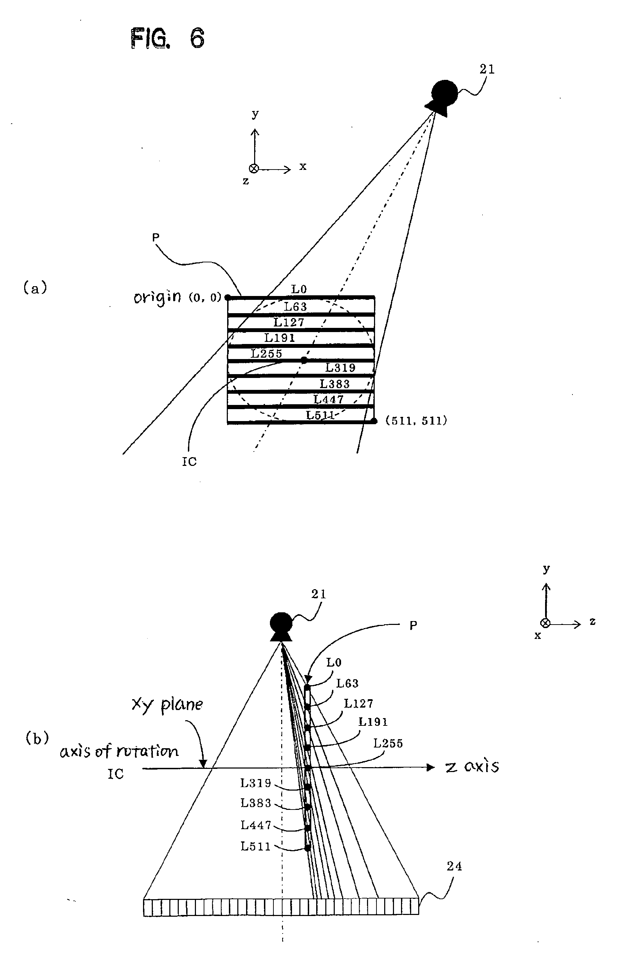

As shown in Fig. 6(a) and Fig. 6(b), a square field having 512 pixel points arranged in rows and in columns and being parallel to the xy plane is regarded as a field of view P. A line of pixel points L0 parallel to an x axis and indicating a y-coordinate of 0, a line of pixel points L63 indicating a y-coordinate of 63, a line of pixel points L127 indicating a y-coordinate of 127, a line of pixel points L191 indicating a y-coordinate of 191, a line of pixel points L255 indicating a y-coordinate of 255, a line of pixel points L319 indicating a y-coordinate of 319, a line of pixel points L383 indicating a y-coordinate of 383, a line of pixel points L447 indicating a y-coordinate of 447, and a line of pixel points L511 indicating a y-coordinate of 511 are taken for instance. Projection data items forming lines T0 to T511 produced by projecting the lines of pixel points L0 to L511 on the surface of the multi-array X-ray detector 24 in the direction of X-ray transmission are regarded as projection data items Dr(view,x,y) representing the lines of pixel points L0 to L511. Herein, x and y values correspond to an x-coordinate and a y-coordinate representing the position of each pixel contained in tomographic image data.

The direction of X-ray transmission is determined with the geometric positions of the focal spot in the X-ray tube 21, each pixel point, and the multi-array X-ray detector 24. Since the z-coordinate z(view) contained in each X-ray detector data D0(view,j,i) is provided as a table position in the z direction of rectilinear movement Ztable(view), even if the X-ray detector data D0(view,j,i) is acquired during acceleration or deceleration, the direction of X-ray transmission can be accurately calculated relative to a geometric data acquisition system including the focal spot and multi-array X-ray detector.

Part of a line may come out of the multi-array X-ray detector 24 in the direction of channels in the same manner as, for example, part of the line T0 produced by projecting the line of pixel points L0 on the surface of the multi-array detector 24 in the direction of X-ray transmission does. In this case, projection data items Dr(view,x,y) forming the line are set to 0s. If part of a line comes out in the z direction, missing projection data items Dr(view,x,y) are interpolated.

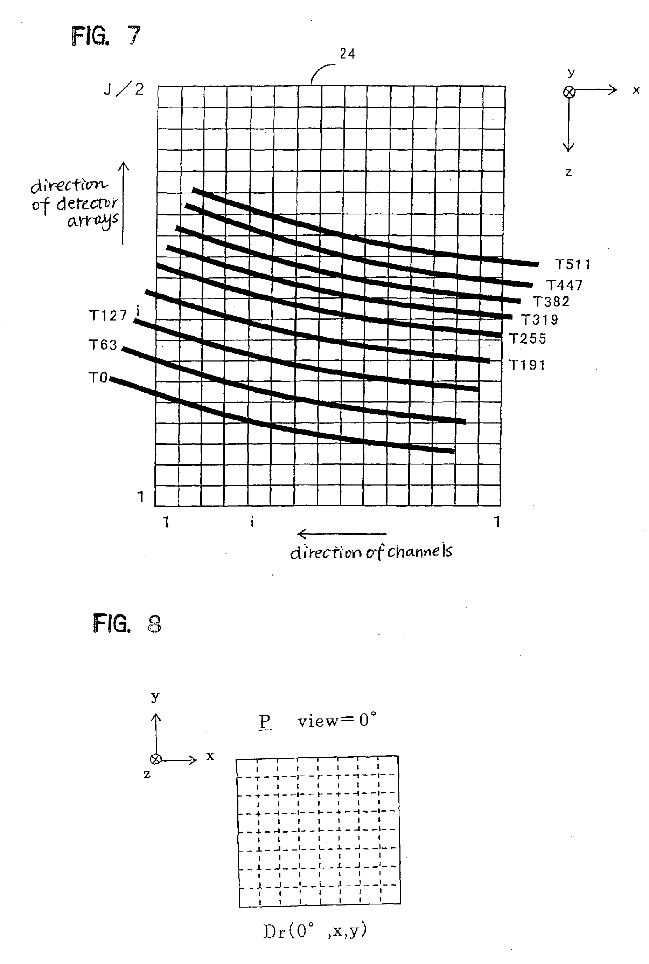

Thus, projection data items Dr(view,x,y) representing the pixel points in the field of view P can be sampled as shown in Fig. 8.

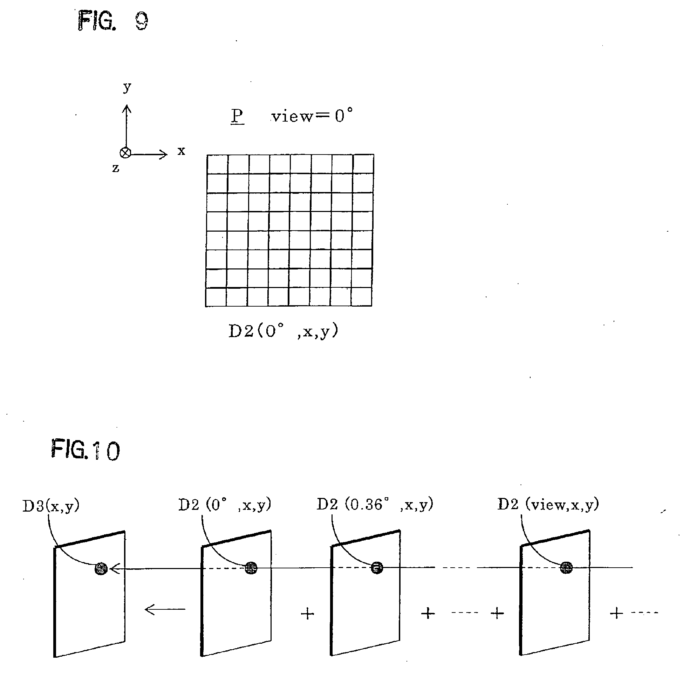

Referring back to Fig. 5, at step S62, the projection data items Dr(view,x,y) are multiplied by either of cone-beam reconstruction weighting coefficients in order to produce projection data items D2(view,x,y) shown in Fig. 9.

The cone-beam reconstruction weighting coefficients $w(i,j)$ will be described below. In case of fan-beam image reconstruction, assuming that $\gamma$ denotes an angle at which a straight line linking the focal spot in the X-ray tube 21 located at a view angle view of $\beta a$ and a pixel point $g(x,y)$ in the field of view (xy plane) meets the center axis Bc of an X-ray beam, and $\beta b$ denotes an opposite view angle view, the opposite view angle $\beta b$ is provided as $\beta a+180°-2\gamma$.

Assuming that $\alpha a$ and $\alpha b$ denote angles at which an X-ray beam passing through the pixel point $g(x,y)$ in the field of view P and an opposite X-ray beam meet the field of view P, cone-beam reconstruction weighting coefficients $\omega a$ and $\omega b$ depend on the angles. Back projection pixel data items D2(0,x,y) are calculated by multiplying the projection data items by either of the cone-beam reconstruction weighting coefficients to perform summating according to the formula (8).

$$D2(0,x,y) = \omega a \cdot D2(0,x,y)\_a + \omega b \cdot D2(0,x,y)\_b \qquad (8)$$

Herein, D2(0,x,y)_a denotes projection data items included in a view $\beta a$, and D2(0,x,y)_b denotes projection data items included in a view $\beta b$.

The sum of the cone-beam reconstruction weighting coefficients $\omega a$ and $\omega b$ dependent on opposed beams is a unity, that is, $\omega a+\omega b=1$.

Since projection data items are multiplied by either of the cone-beam reconstruction weighting coefficients $\omega a$ and $\omega b$ and then summated, conical-angle artifacts can be reduced.

For example, the cone-beam reconstruction weighting coefficients $\omega a$ and $\omega b$ may be calculated as described below.

Assuming that $\gamma max$ denotes a half of the angle of a fan beam, the cone-beam reconstruction weighting coefficients $\omega a$ and $\omega b$ may be calculated according to the equations (9), (10), (11), (12), (13), and (14) presented below. Herein, ga denotes a weighting coefficient associated with an X-ray beam, and gb denotes a weighting coefficient associated with an opposite X-ray beam.

$$ga = f(\gamma max,\alpha a,\beta a) \qquad (9)$$

$$gb = f(\gamma max,\alpha b,\beta b) \qquad (10)$$

$$xa = 2 \cdot ga^q / (ga^q+gb^q) \qquad (11)$$

$$xb = 2 \cdot gb^q / (ga^q+gb^q) \qquad (12)$$

$$\omega a = xa^2 \cdot (3-2xa) \qquad\qquad (13)$$

$$\omega b = xb^2 \cdot (3-2xb) \qquad\qquad (14)$$

Herein, for example, q equals 1.

For example, assuming that ga and gb are functions of max[ ] providing a larger one of 0 and $\{(\pi/2+\gamma max)\cdot|\beta a|\}$, ga and gb are rewritten as follows:

$$ga = max[0,\{(\pi/2+\gamma max)\cdot|\beta a|\}] \cdot |tan(\alpha a)| \qquad (15)$$

$$gb = max[0,\{(\pi/2+\gamma max)\cdot|\beta b|\}] \cdot |tan(\alpha b)| \qquad (16)$$

In the case of fan-beam image reconstruction, the projection data items representing the pixel points in the field of view P are multiplied by a distance coefficient. The distance coefficient is provided as $(r1/r0)^2$ where r0 denotes a distance from the focal spot in the X-ray tube 21 to a detector element that belongs to a detector array j and a channel i included in the multi-array X-ray detector 24 and that detects projection data Dr and r1 denotes a distance from the focal spot in the X-ray tube 21 to a pixel point in the field of view P represented by the projection data Dr.

In the case of parallel-ray beam image reconstruction, the projection data items representing the pixel points in the field of view P are multiplied by either of the cone-beam reconstruction weighting coefficients w(i,j) alone.

At step S63, as shown in Fig. 10, projection data items D2(view,x,y) are pixel by pixel added to back projection data items D3(x,y) that are cleared in advance.

At step S64, steps S61 to S63 are repeated for all views required to reconstruct a tomographic image, that is, views produced with the X-ray tube rotated 360° (or 180° + the angle of a fan-shaped beam) so as to produce back projection data items D3(x,y) as shown in Fig. 10.

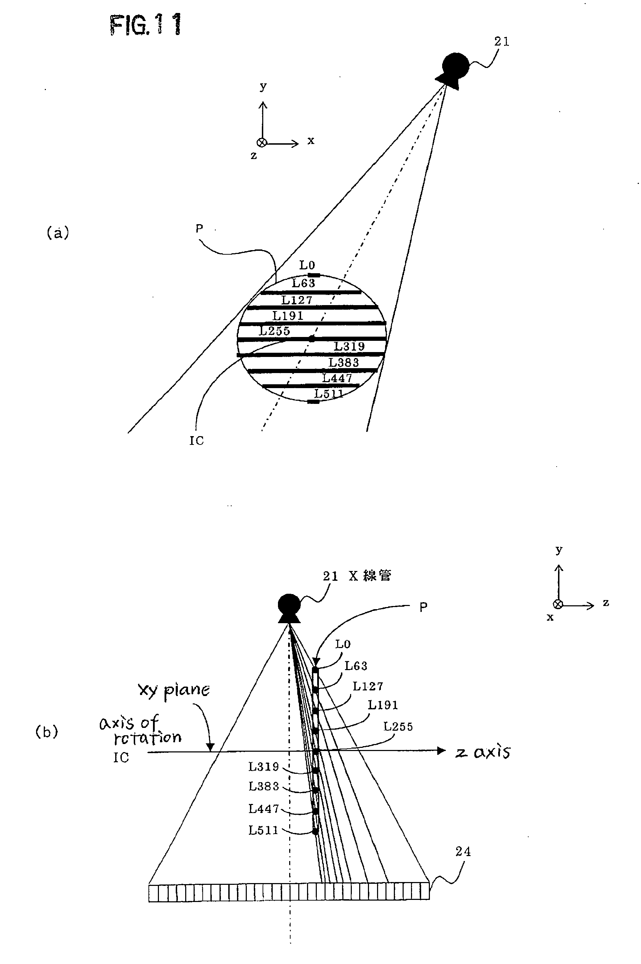

As shown in Fig. 11 (a) and Fig. 11 (b), the field of view P may not be square but may be a circular field whose diameter corresponds to 512 pixels.

Consequently, the X-ray CT apparatus performs the foregoing image reconstruction by following the steps described below so as to thus reconstruct each tomographic image.

At step S1, data acquisition is performed.

At step S2, preprocessing is performed.

At step S3, beam hardening compensation is performed.

At step S4, z filter convolution is performed.

At step S5, reconstruction function convolution is performed.

At step S6, three-dimensional back projection is performed.

At step S7, post-processing is performed.

The image reconstruction for reconstructing a tomographic image is repeatedly performed in order to reconstruct tomographic images expressing sections of a subject that succeed in the z direction, whereby a three-dimensional image composed of the successive tomographic images expressing the sections succeeding in the z direction is produced.

Subsequent time-sequential three-dimensional spatial filtering of step S8 will be described below.

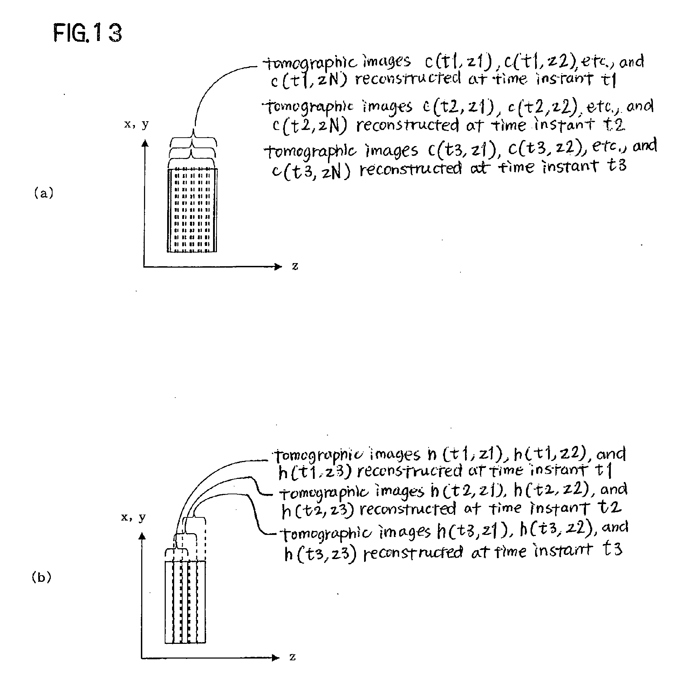

Fig. 13 shows time-sequential helical-scan and cine-scan images produced at step S7.

In the X-ray CT apparatus employing the multi-array X-ray detector 24, when helical scanning is adopted and three-dimensional image reconstruction is performed, not only one tomographic image $h(t_1,z)$ but also a plurality of, that is, N tomographic images $h(t_1,z_1)$, $h(t_1,z_2)$, etc., $h(t_1, z_{N-1})$, and $h(t_1,z_N)$ can be produced. In this case, images $h(t_i,z_i)$ and $h(t_k,z_1)$ may be reconstructed so that they will express the same section of a subject. In other words, a plurality of tomographic images reconstructed at different time instants may express the same section whose position is represented by the same z-coordinate.

Fig. 13(a) shows images constructed by adopting cine scanning. Herein, one rotational data acquisition or a plurality of rotational data acquisitions is performed at a certain position in the z direction. Consequently, time-sequential tomographic images listed below are reconstructed.

Tomographic images $c(t_1,z_1)$, $c(t_1,z_2)$, etc., $c(t_1,z_{N-1})$, and $c(t_1,z_N)$ reconstructed at time instant $t_1$

Tomographic images $c(t_2,z_1)$, $c(t_2,z_2)$, etc., $c(t_2,z_{N-1})$, and $c(t_2,z_N)$ reconstructed at time instant $t_2$

Tomographic images $c(t_3,z_1)$, $c(t_3,z_2)$, etc., $c(t_3,z_{N-1})$, and $c(t_3,z_N)$ reconstructed at time instant t3

Tomographic images $c(t_M,z_1)$, $c(t_M,z_2)$, etc., $c(t_M,z_{N-1})$, and $c(t_M,z_N)$ reconstructed at time instant tM

When the tomographic images $c(t,z_1)$, $c(t,z_2)$, etc., $c(t,z_{N-1})$, and $c(t,z_N)$ are combined in the order that the sections expressed thereby lie in the z direction, a three-dimensional image Cine3D(t) expressing the state of a subject attained at a certain time instant is produced. Thus, three-dimensional images expressing the states of the subject attained at time instants $t_1$ to tM are produced. The three-dimensional images shall be called time-sequential three-dimensional images.

In order to detect a time-varying change in a tomographic image expressing a section of a subject located at a certain position in the z direction, tomographic images $c(t_1,z_i)$, $c(t_2,z_i)$, $c(t_3,z_i)$, etc., and $c(t_M,z_i)$ are sampled as time-sequential tomographic images.

Fig. 13(b) shows images reconstructed by adopting helical scanning. The following tomographic images are reconstructed time-sequentially:

Tomographic images $h(t_1,z_1)$, $h(t_1,z_2)$, etc., $h(t_1,z_{N-1})$, and $h(t_1,z_N)$ reconstructed at time instant $t_1$

Tomographic images $h(t_2,z_2)$, $h(t_2,z_3)$, etc., $h(t_2,z_N)$, and $h(t_2,z_{N+1})$ reconstructed at time instant $t_2$

Tomographic images $h(t_3,z_3)$, $h(t_3,z_4)$, etc., $h(t_3,z_{N+1})$, and $h(t_M,z_{N+2})$ reconstructed at time instant $t_3$

Tomographic images $h(t_M,z_M)$, $h(t_M,z_{M+1})$, etc., $h(t_M,z_{N+M-2})$, and $h(t_3,z_{N+M-1})$ reconstructed at time instant $t_M$

In order to detect a time-varying change in a tomographic image expressing a section of a subject located at a certain position in the z direction, time-sequential tomographic images $h(t_1,z_N)$, $h(t_2,z_N)$, $h(t_3,z_N)$, etc. are sampled.

Assuming that M<N is established, when tomographic images $h(t,z_M)$, $h(t,z_{M+1})$, etc., and $h(t,z_N)$ are combined in the order that the sections expressed thereby lie in the z direction, a three-dimensional image Helical3D(t) expressing a range of a subject from a z-coordinate $z_M$ to a z-coordinate $z_N$ is produced. Thus, three-dimensional images expressing the range of the subject at time instants $t_1$ to $t_M$ are produced as time-sequential three-dimensional images.

Three-dimensional image composed of tomographic images $h(t_1,z_M)$, $h(t_1,Z_{M+1})$, etc., and $h(t_1,Z_N)$

Three-dimensional image composed of tomographic images $h(t_2,z_M)$, $h(t_2,Z_{M+1})$, etc., and $h(t_2,Z_N)$

Three-dimensional image composed of tomographic images $h(t_M,z_M)$, $h(t_M,Z_{M+1})$, etc., and $h(t_M,Z_N)$

When a helical pitch is smaller than 1, a range of a subject expressed by a group of tomographic images reconstructed at one time instant, which extends in the z direction, overlaps, as shown in Fig. 13(b), a range of the subject expressed by another group of tomographic images, which are reconstructed at another time instant, to a great extent.

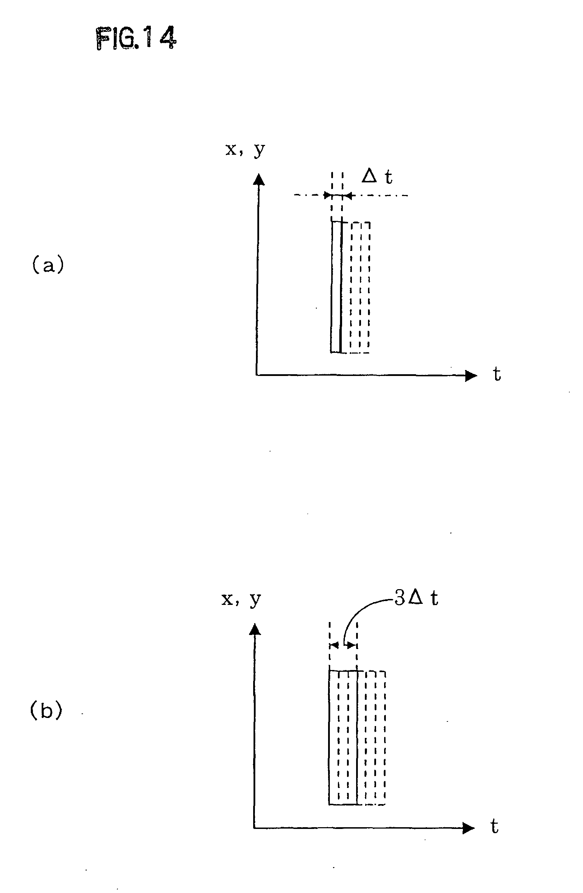

Moreover, whichever of helical scanning and cine scanning is adopted, a slice thickness may correspond to an inter-tomographic image spacing as shown in Fig. 14(a). Otherwise, the slice thickness may be, as shown in Fig. 14 (b), larger than the inter-tomographic image spacing in order to reduce noises in an image.

At step S8 in Fig. 3, four-dimensional spatial filtering is performed on time-sequential three-dimensional images produced through cine scanning or helical scanning.

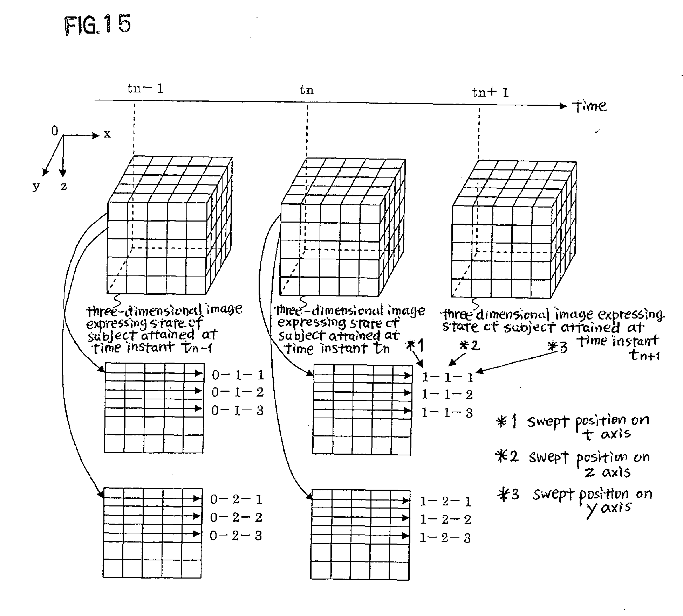

Fig. 15 is an explanatory diagram concerning convolution of a four-dimensional spatial filter to time-sequential three-dimensional images that are three-dimensional images produced at time instants $t_{n-1}$, $t_n$, and $t_{n+1}$ and that constitute a four-dimensional image.

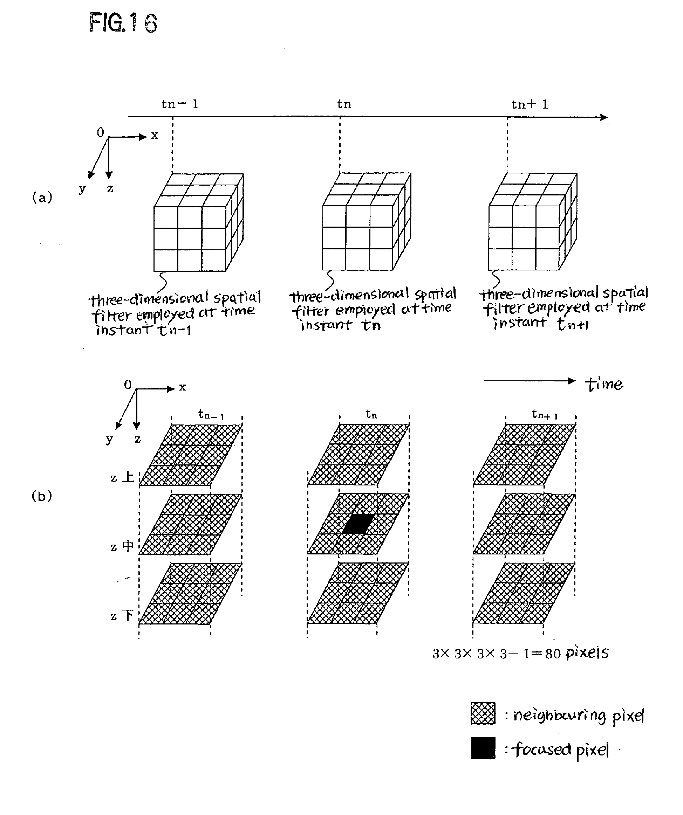

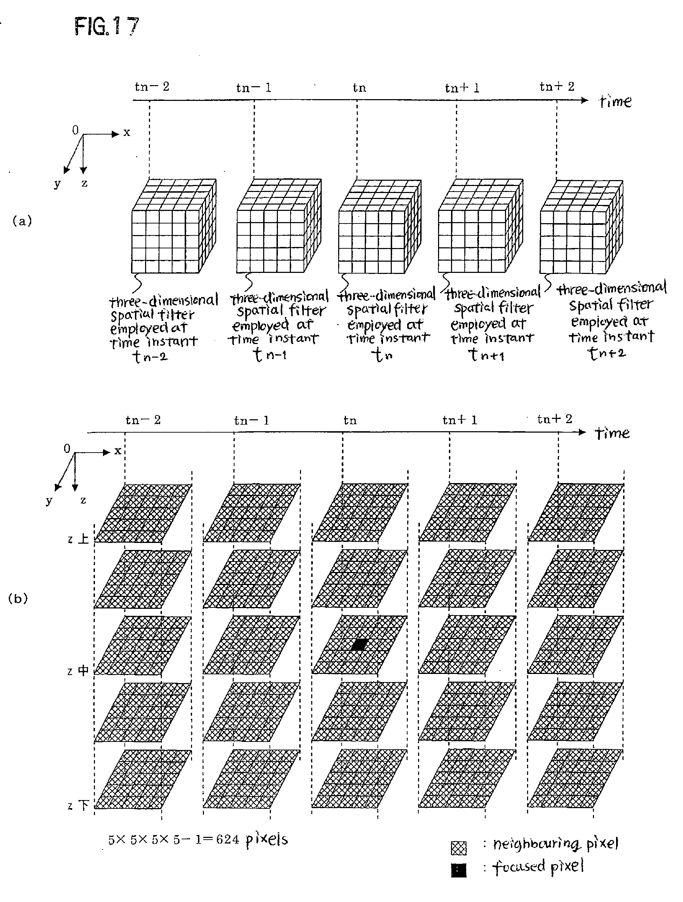

As examples of a four-dimensional spatial filter, Fig. 16 shows a four-dimensional spatial filter that has three filtering coefficients defined in four dimensions and that is applied to a neighborhood composed of eighty neighbors, and Fig. 17 shows a four-dimensional spatial filter that has five filtering coefficients defined in four dimensions and that is applied to a neighborhood composed of six hundreds and twenty-four neighbors. Fig. 16(a) and Fig. 17(b) are conceptual diagrams showing the four-dimensional spatial filters. Fig. 16(b) and Fig. 17(b) are conceptual diagrams showing a focused pixel and neighboring pixels that are selected when each of the four-dimensional spatial filters is used to perform three-dimensional spatial filtering on each of three-dimensional images that are arrayed time-sequentially and produced at respective time instants.

As shown in Fig. 15, when a four-dimensional spatial filter is convoluted to a three-dimensional image produced at a time instant $t_n$, processing described below is performed.

For example, when a four-dimensional spatial filter having three filtering coefficients defined in four dimensions is convoluted, resultant three-dimensional images are expressed by the formula (17) presented below. Incidentally, a three-dimensional spatial filter to be convoluted to each three-dimensional image produced at each time instant has three filtering coefficients defined in three dimensions as shown in Fig. 16. In the formula below, an asterisk * denotes convolution.

Three-dimensional images produced at time instants $t_n$ and subjected to four-dimensional spatial filter convolution = (three-dimensional image produced at time instant $t_{n-1}$) * (three-dimensional spatial filter employed at time instant $t_{n-1}$) + (three-dimensional image produced at time instant $t_n$) * (three-dimensional spatial filter employed at time instant $t_n$) + (three-dimensional image produced at time instant $t_{n+1}$) * (three-dimensional spatial filter employed at time instant $t_{n+1}$)  (17)

When a four-dimensional spatial filter having five filtering coefficients defined in four dimensional is convoluted, resultant three-dimensional images are expressed by the formula (18) presented below. Incidentally, a three-dimensional spatial filter to be convoluted to a three-dimensional image produced at each time instant has five filtering coefficients defined in three dimensions as shown in Fig. 17.

(Three-dimensional images produced at time instants $t_n$ and subjected to four-dimensional spatial filter convolution) = (three-dimensional image produced at time instant $t_{n-2}$) * (three-dimensional spatial filter employed at time instant $t_{n-2}$) + (three-dimensional image produced at time instant $t_{n-1}$) * (three-dimensional spatial filter employed at time instant $t_{n-1}$) + (three-dimensional image produced at time instant $t_n$) * (three-dimensional spatial filter employed at time instant $t_n$) + (three-dimensional image produced at time instant $t_{n+1}$) * (three-dimensional spatial filter employed at time instant $t_{n+1}$) + (three-dimensional image produced at time instant $t_{n+2}$) * (three-dimensional spatial filter employed at time instant $t_{n+2}$)

(18)

Moreover, when a spatial filter is convoluted, the spatial filter is, as shown in Fig. 15, swept through pixels one by one. The sets of three numerals presented below indicate the positions of pixels each of which is represented by a swept position in a t axis, a swept position in a z axis, and a swept position on a y axis.

0-1-1 → 0-1-2 → 0-1-3 → ... → 0-2-1 → 0-2-2 → 0-2-3 → ... → ... → 1-1-1 → 1-1-2 → 1-1-3 → ... → 1-2-1 → 1-2-2 → 1-2-3 → ... → ...

Consequently, a four-dimensional spatial filter is convoluted to three-dimensional images produced at time instants $t_n$. When the four-dimensional spatial filter is convoluted to time-sequential three-dimensional images, the four-dimensional spatial filter is convoluted not only to the three-dimensional images produced at the time instants $t_n$ but also to images produced over a required time interval including time instants $t_1$, $t_2$, etc., $t_{n-1}$, $t_{n+1}$, etc., and $t_N$.

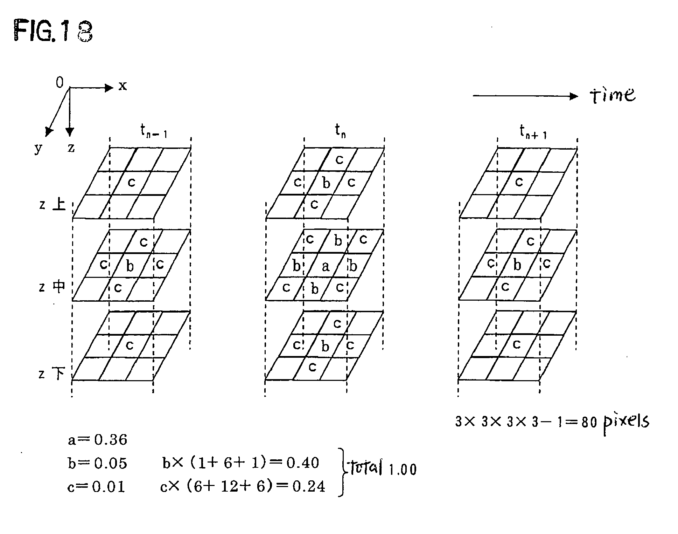

Fig. 18 shows an example of a four-dimensional spatial filter having three filtering coefficients defined in four dimensions and being intended to reduce noises. In Fig. 18, a, b, and c denote spatial filtering coefficients by which respective pixel values are multiplied. For example, a is set to 0.36, b is set to 0.05, and c is set to 0.01.

In this case, as shown in Fig. 18, the value of a focused pixel produced at a time instant tn is multiplied by the spatial filtering coefficient a of 0.36. The values of pixels neighboring the focused pixel in the x, y, and z directions and being produced at the same time instant $t_n$ are multiplied by the spatial filtering coefficient b of 0.05. Moreover, the values of pixels being produced at the same time instant $t_n$ as the focused pixel is and immediately neighboring the focused pixels in directions that meet the x and y directions at 45° on the xy plane and in directions that meet the x and z directions at 45° on the xz plane are multiplied by the spatial filtering coefficient c of 0.01. Furthermore, the values of pixels that are located at the same position in the xyz space as the focused pixel produced at the time instant $t_n$ and that are produced at a time instant $t_{n-1}$ immediately preceding the time instant $t_n$ and at a time instant $t_{n+1}$ immediately succeeding the time instant $t_n$ are multiplied by the spatial filtering coefficient b of 0.05. The values of pixels that are produced at the time instant $t_{n-1}$ preceding the time instant $t_n$ and at the time instant $t_{n+1}$ succeeding the time $t_n$, and that neighbor the same position in the xyz space as the position of the focused pixel in the x, y, and z directions are multiplied by the spatial filtering coefficient c of 0.01. Thereafter, the products of the pixels by the spatial filtering coefficients are summated, and the sum total is regarded as the value of the focused pixel.

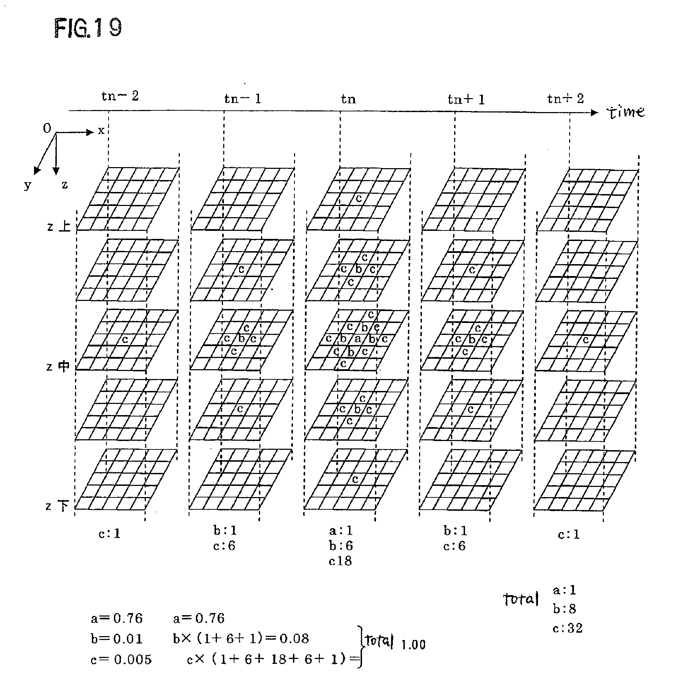

Fig. 19 shows an example of a four-dimensional spatial filter having five filtering coefficients defined in four dimensions and being intended to reduce noises. In Fig. 19, a, b, and c denote spatial filtering coefficients by which the values of respective pixels are multiplied, and are set to, for example, 0.76, 0.01, and 0.005 respectively.

In this case, as shown in Fig. 19, the value of a focused pixel produced at a time instant $t_n$ is multiplied by the spatial filtering coefficient a of 0.76. The values of pixels immediately neighboring the focused pixel in the x, y, and z directions and being produced at the same time instant $t_n$ as the focused pixel is are multiplied by the spatial filtering coefficient b of 0.01. The values of pixels neighboring the pixels that immediately neighbor the focused pixel in the x, y, and z directions respectively are multiplied by the spatial filtering coefficient b of 0.005. Moreover, the values of pixels that are produced at the same time instant $t_n$ as the focused pixel is and that immediately neighbor the focused pixel in directions which meet the x and y directions at 45° on the xy plane and in directions which meet the x and z directions at 45° on the xz plane are multiplied by the spatial filtering coefficient c of 0.005. Furthermore, the values of pixels that are located at the same position in the xyz space as the focused pixel produced at the time instant $t_n$, and that are that are produced at a time instant $t_{n-1}$ immediately preceding the time instant $t_n$ and at a time instant $t_{n+1}$ immediately succeeding the time instant $t_n$ are multiplied by the spatial filtering coefficient b of 0.01. Furthermore, the values of pixels that are produced at the time instant $t_{n-1}$ preceding the time instant $t_n$ and at the time instant $t_{n+1}$ succeeding the time instant $t_n$ and that immediately neighbor the same position in the xyz space as the position of the focused pixel in the x, y, and z directions respectively are multiplied by the spatial filtering coefficient c of 0.005. The values of pixels that are located at the same position in the xyz space as the position of the focused pixel produced at the time instant $t_n$, and that are produced at a time instant $t_{n-2}$ preceding the time instant immediately preceding the time instant $t_n$ and at a time instant $t_{n+2}$ succeeding the time instant immediately succeeding the time instant $t_n$ are multiplied by the spatial filtering coefficient c of 0.005. Thereafter, the products of the pixels by the spatial filtering coefficients are summated, and the sum total of the products is regarded as the value of the focused pixel.

The spatial filters included in the four-dimensional spatial filter and intended to reduce noises are passive filters that implement specific spatial filtering whatever a focused pixel is and whatever pixels neighbor the focuses pixel.

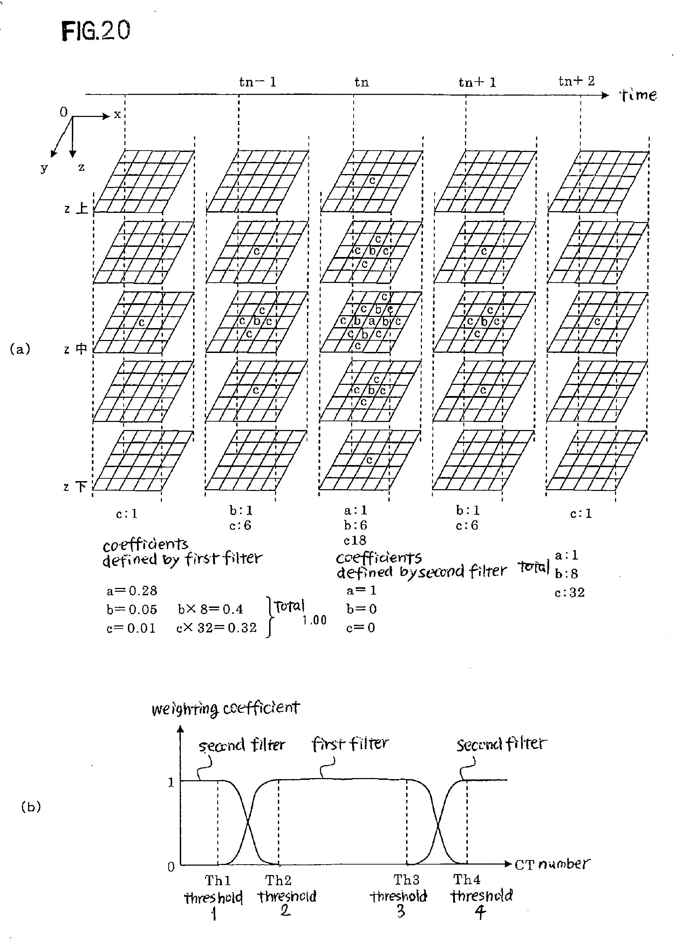

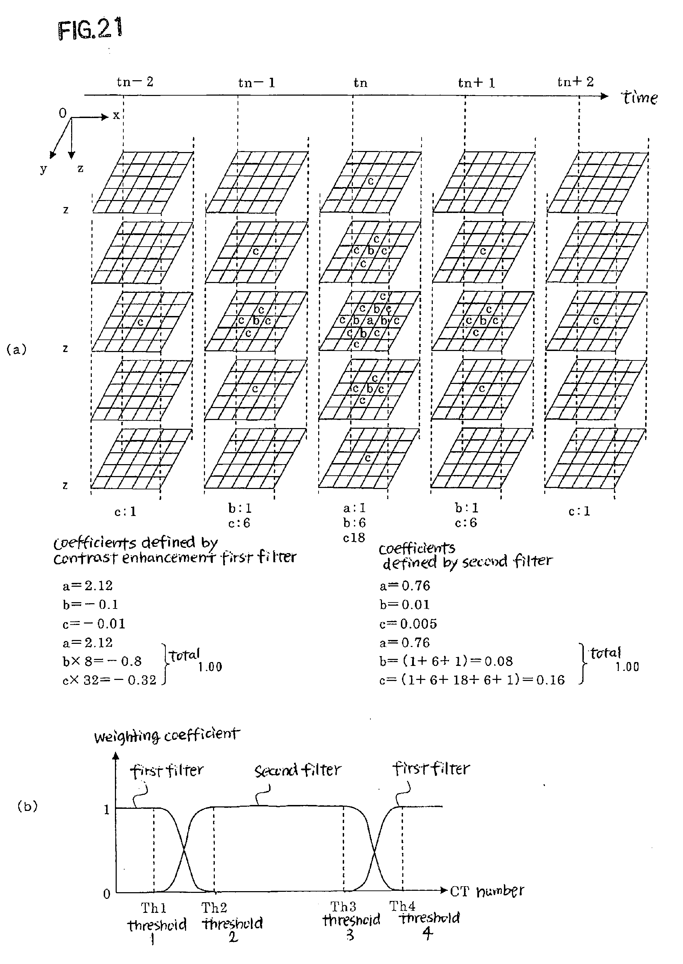

Fig. 20 shows an example of a four-dimensional spatial filter intended to reduce noises and dependent on a CT number, and Fig. 21 shows an example of the four-dimensional spatial filter intended to reduce noises and enhance a contrast and dependent on a CT number. In spatial filtering implemented by each of the four-dimensional spatial filter, spatial filtering coefficients to be convoluted vary depending on a CT number. Fig. 20(a) and Fig. 21 (a) show scenes of filtering of pixels contained in three-dimensional image data. Herein, a, b, and c denote spatial filtering coefficients by which the values of pixels are multiplied. Fig. 20(b) and Fib. 21(b) graphically show the relationship of the sum totals of spatial filtering coefficients included in first and second filters, which are used for filtering, to CT numbers represented by respective pixels.

In the X-ray CT apparatus, spatial filtering varies depending on the value of a focused pixel that is a CT number or depending on what kind of image is expressed by the pixel. In other words, filters to be convoluted are switched based on what tissue in a region is expressed by the focused pixel, for example, based on whether the focused pixel expresses a soft tissue, a bone tissue, or a tissue in the lung field. In general, when the focused pixel expresses the soft tissue, since smoother image quality is requested, a spatial filter intended to reduce noises is adopted. When the focused tissue expresses the bone tissue or the tissue in the lung field, since a fine structure is requested to be visualized, a spatial filter intended to enhance a contrast or enhance a high-frequency component is adopted.

Specifically, as shown in Fig. 20(a), a first filter whose spatial filtering coefficients a, b, and c shown in Fig. 19 assume 0.28, 0.05, and 0.01 respectively and a second filter whose spatial filtering coefficients, a, b, and c assume 1, 0, and 0 respectively are employed. As shown in Fig. 20(b), the spatial filtering coefficients are switched from those defined by the first filter to those defined by the second filter or vice versa according to a CT number. Thus, the first and second filters are switched.

Likewise, as shown in Fig. 21 (a), a first filter whose spatial filtering coefficients a, b, and c shown in Fig. 19 assume 2.12, -0.1, and -0.01 respectively and a second filter whose spatial filtering coefficients a, b, and c assume 0.76, 0.01, and 0.005 respectively are employed. As shown in Fig. 21(b), the spatial filtering coefficients are switched from those defined by the first filter to those defined by the second filter or vice versa according to a CT number. Thus, the first and second filters are switched.

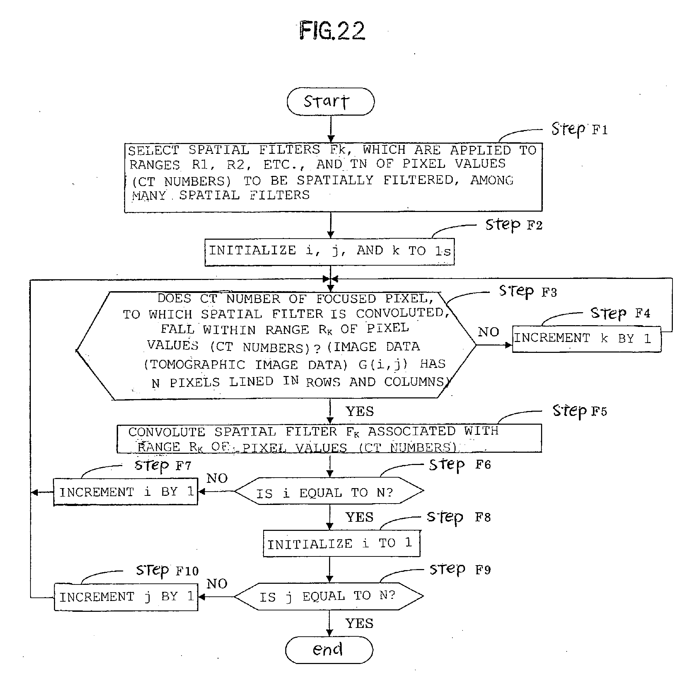

Fig. 22 is a flowchart describing spatial filtering dependent on a CT number.

At step F1, spatial filters Fk to be convoluted to certain ranges of pixel values (CT numbers) $R_1$, $R_2$, etc., and RN are selected from among a plurality of spatial filters. At this time, the ranges of pixel values (CT numbers) $R_1$, $R_2$, etc., and $R_N$ should cover all CT numbers but not overlap. Namely, the ranges are determined as expressed by the formulae (19) and (20) presented below. However, $R_K$ denotes each range of CT numbers indicated with a lower limit of the CT numbers in the range $R_K$ and an upper limit thereof.

$$R_1 \cap R_2 \cap \quad \ldots\ldots \quad \cap R_N = \phi \text{ (null set)} \tag{19}$$

$$R_1 \cup R_2 \cup \quad \ldots\ldots \quad \cup R_N = \text{sum of sets} \tag{20}$$

At step F2, i, j, and k values are initialized to 1s. Thus, initialization is performed for spatial filtering. Herein, i denotes an x-coordinate indicating the position of a pixel contained in tomographic image data, j denotes a y-coordinate indicating the position of the pixel, and k denotes a number assigned to a range of CT numbers.

At step F3, a CT number represented by a focused pixel G(i,j) to be spatially filtered is checked to see if it falls within a range $R_K$ of pixel values (CT numbers) (image data (tomographic image data) G(i,j) has N pixels lined in rows and columns). If so (Yes), control is passed to step F5. Otherwise (No), control is passed to step F4.

At step F4, the k value is incremented by one, that is, is set to k+1. Control is then returned to step F3.

At step F5, a spatial filter $F_K$ associated with the range $R_K$ of pixel values (CT numbers) is convoluted to the pixel values.

At step F6, the i value is checked to see if it equals N. If so (Yes), control is passed to step F8. Otherwise (No), control is passed to step F7. Herein, N denotes the size of tomographic image data indicated by the number of pixels.

At step F7, the i value is incremented by one, that is, is set to i+1. Control is then returned to step F3.

At step F8, the i value is set to 1.

At step F9, the j value is checked to see if it equals N. If so (Yes), the processing is terminated. Otherwise (No), control is passed to step F10.

At step F10, the j value is incremented by one, that is, is set to j+1. Control is then returned to step F3.

When time-sequential three-dimensional spatial filtering or four-dimensional spatial filtering is adopted as spatial filtering, appropriate spatial filtering can be effectively performed depending on a range of CT numbers.

Spatial filtering dependent on the value of a focused pixel has been described so far. When the spatial filtering is performed in consideration of not only the property of the focused pixel but also the properties of neighboring pixels, the spatial filtering can be performed more appropriately.

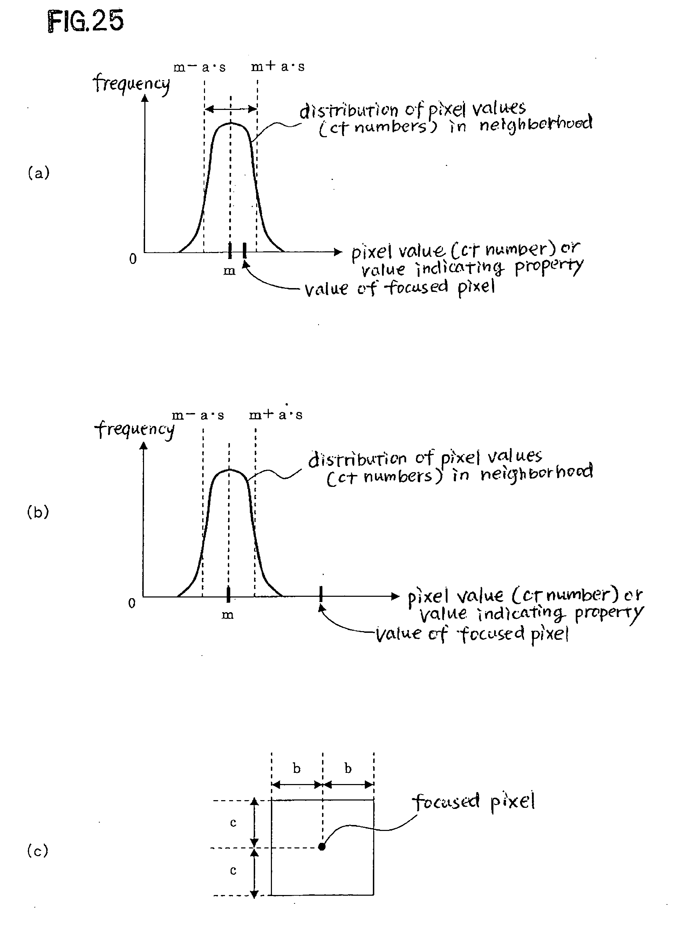

Fig. 25(a) and Fig. 25(b) each show a histogram indicating the values of pixels belonging to a neighborhood of a focused pixel and the relationship of the value of the focused pixel to the values of the pixels belonging to the neighborhood. In Fig. 25(a) and Fig. 25(b), m denotes a mean of the values of the pixels belonging to the neighborhood, s denotes a standard deviation of any of the pixel values included in the neighborhood, and a denotes a constant. Herein, CT numbers are adopted as an example expressing a property of image data. The same applies to any other value expressing the property of image data.

As shown in Fig. 25(c), when coordinates indicating the position of a focused pixel are coordinates (i,j), a range defined with x-coordinates ranging from i-b to i+b and y-coordinates ranging from j-c to j+c shall be regarded as a neighborhood.

Fig. 25(a) is concerned with a case where the property of the neighborhood resembles that of the focused pixel, while Fig. 25(b) is concerned with a case where the property of the neighborhood does not resemble that of the focused pixel.

In Fig. 25(a), the value of the focused pixel falls within a range from (m-a·s) to (m+a.s) where m denotes a mean of the values of pixels belonging to the neighborhood and s denotes a standard deviation of each of the pixel values in the neighborhood. In Fig. 25(b), the value of the focused pixel does not fall within the range from (m-a·s) to (m+a.s) where m denotes a mean of the values of pixels belonging to the neighborhood and s denotes a standard deviation of each of the pixel values in the neighborhood.

In other words, in Fig. 25(a), the property of the focused pixel resembles the property of the neighborhood. However, in Fig. 25(b), the property of the focused pixel does not resemble that of the neighborhood. Namely, the focused pixel is recognized as a pixel having a different property.

Whether the property of a focused pixel resembles that of a neighborhood is determined based on the foregoing criterion. Consequently, a spatial filter suitable for each case can be applied properly.

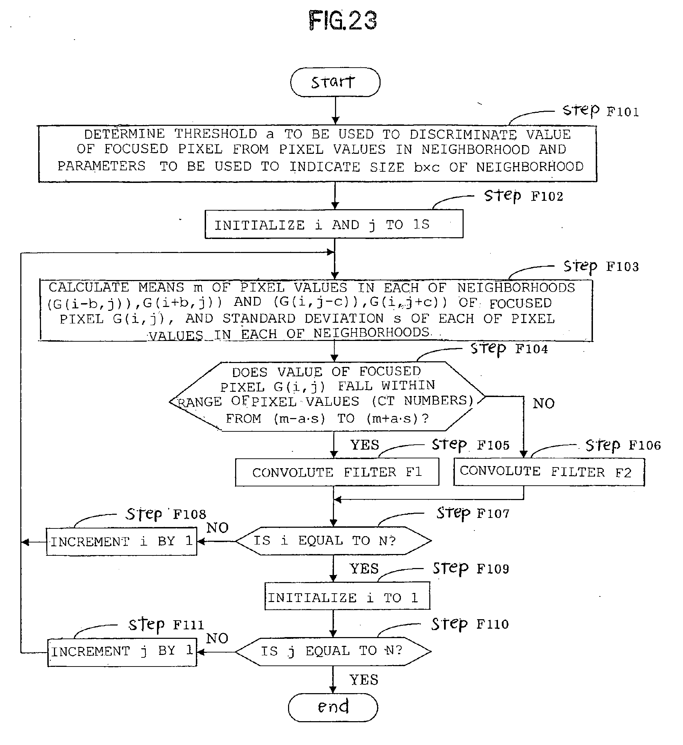

Fig. 23 is a flowchart describing spatial filtering which depends on the property of a neighborhood and to which the foregoing idea is applied.

At step F101, a threshold a to be used to discriminate the value of a focused pixel from that of a neighboring pixel, and parameters to be used to indicate the size b×c of a neighborhood are determined. Thus, the parameters required for the spatial filtering are designated.

At step F102, i and j values are initialized to 1s. Thus, initialization for the spatial filtering is achieved.

At step F103, a mean m of pixel values in each of neighborhoods (G(i-b,j),G(i+b,j)) and (G(i,j-c),G(i,j+c)) of a focused pixel G(i,j) and a standard deviation s of each of the pixel values in each of the neighborhoods are calculated.

At step F104, the value of the focused pixel G(i,j) is checked to see if it falls within a range from a pixel value (CT number) (m-a·s) to a pixel value (m+a.s). If so (Yes), control is passed to step F105. Otherwise (No), control is passed to step F106.

At step F105, a filter F1 is convoluted. Thereafter, control is passed to step F107.

At step F106, a filter F2 is convoluted. Thereafter, control is passed to step F107.

At step F107, the value i is checked to see if it equals N. If so (Yes), control is passed to step F109. Otherwise (No), control is passed to step F108.

At step F108, the value i is incremented by one, that is, is set to i+1. Control is then returned to step F103.

At step F109, the value i is initialized to 1.

At step F110, the value j is checked to see if it equals N. If so (Yes), the processing is terminated. Otherwise (No), control is passed to step F111.

At step F111, the value j is incremented by one, that is, is set to j+1. Control is then returned to step F103.

When time-sequential three-dimensional spatial filtering or four-dimensional spatial filtering is adopted as the spatial filtering, spatial filtering can be effectively performed depending on the property of a neighborhood.

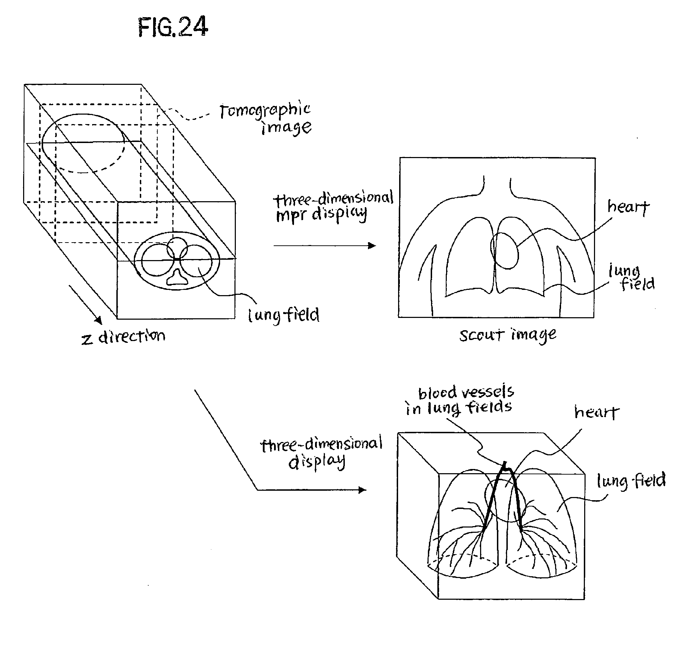

At step S9 in Fig. 3, four-dimensional spatial filtering is performed in order to reduce noises or enhance a contrast, and a resultant tomographic image is displayed. Otherwise, as shown in Fig. 24, three-dimensional MPR display or three-dimensional display is performed. The MPR display is a display method of displaying a three-dimensional image, which is composed of a plurality of tomographic images, with a zx plane a zy plane, or any other oblique plane converted.

According to the present embodiment, pieces of information on the directions of a time axis and all spatial axes are used to improve the quality of a four-dimensional image that is a time-varying three-dimensional image and composed of time-sequential three-dimensional images, a three-dimensional image that is a time-varying two-dimensional image and composed of time-sequential two-dimensional images, or an N-dimensional image that is a time-varying N-1-dimensional image and composed of time-sequential N-1-dimensional images.

According to the present embodiment, pieces of information on the directions of a time axis and spatial axes are used to improve the quality of time-sequential three-dimensional images or time-sequential two-dimensional images produced through conventional (axial) scanning, cine scanning, or helical scanning performed by an X-ray CT appàratus including a matrix-type two-dimensional area X-ray detector represented by a multi-array X-ray detector or a flat-panel X-ray detector. Consequently, target image quality can be realized with a smaller X-ray dose.

As mentioned above, the X-ray CT apparatus 100 in accordance with the present embodiment reconstructs a three-dimensional image, which is composed of tomographic images of a subject, sequentially in the direction of a time axis according to projection data items produced by scanning the subject with X-rays. In the present embodiment, the central processing unit 3 spatially filters three-dimensional images that are successively produced in the direction of the time axis, and also filters them in the direction of the time axis. For example, filtering intended to remove noises or enhance a contrast is performed in the directions of spatial axes and the direction of the time axis. On the monitor 6, the three-dimensional images filtered by the central processing unit 3 are successively displayed in association with time instants at which they are produced. Since filtering is performed even in the direction of the time axis, the present embodiment can improve the quality of three-dimensional images that are successively produced in the direction of the time axis.

Moreover, in the present embodiment, the central processing unit 3 can perform a plurality of kinds of filtering. Any of the plurality of kinds of filtering is sequentially selected and performed on a focused pixel contained in reconstructed three-dimensional image data according to the value of the focused pixel. For example, any of the plurality of kinds of filtering is sequentially selected based on the value of the focused pixel. Otherwise, any of the plurality of kinds of filtering is sequentially selected based on a difference of the value of the focused pixel from the value of a pixel neighboring the focused pixel. Thereafter, the central processing unit 3 successively performs the selected kinds of filtering on focused pixels contained in respective three-dimensional image data items that are successively produced in the direction of time axis. For example, when the value of the focused pixel falls within a predetermined range, the pixel value is provided as it is. When the value of the focused pixel falls outside the predetermined range, the value of the focused pixel having undergone filtering intended to remove noises is provided as a pixel value. Consequently, according to the present embodiment, the quality of three-dimensional images successively reconstructed in the direction of a time axis can be improved.

Noted is that the present invention is not limited to the aforesaid embodiment but various variants can be adopted.

For example, a three-dimensional image reconstruction method based on a known Feldkamp method may be adopted or any other three-dimensional reconstruction method may be adopted.

In the aforesaid embodiment, a filter having different coefficients defined in the direction of detector arrays (z direction) is convoluted to image data items in order to adjust a variation in image quality among image data items produced by the detector arrays. Consequently, a slice thickness and image quality susceptible to artifacts or noises are realized to be uniform over the data items produced by the detector arrays. Various sets of filtering coefficients are conceivable and would prove equally effective.

The present invention can be applied not only to an X-ray CT apparatus for medical purposes but also to an X-ray CT apparatus for industrial purposes or a combination of the X-ray CT apparatus with any other modality, such as; an X-ray CT-PET system or an X-ray CT-SPECT apparatus.

In the example of spatial filtering dependent on a pixel value, a noise reduction filter and a contrast enhancement filter have been employed. Alternatively, a plurality of spatial filters having any other capabilities may be employed and would prove effective. As the noise reduction filter and contrast enhancement filter, sets of coefficients to be defined by one spatial filter have been take for instance. Needless to say, any other sets of coefficients would also prove effective.

In the example of spatial filtering dependent on the property of a neighborhood, a mean and a standard deviation which are employed in a statistical technique are used to determine whether the property of a focused pixel resembles that of a neighborhood. Even when any other method is adopted, as long as the property of the focused pixel is compared with that of a neighborhood and a determination is made based on a certain criterion, the same advantage as the aforesaid one would be provided.

The embodiment has been described in relation to time-sequential three-dimensional images produced through cine scanning or helical scanning. Even when tomographic images are produced at regular intervals through conventional (axial) scanning, the same advantage as the aforesaid one would be provided.

Fig. 20, Fig. 21, and Fig. 22 show examples of a four-dimensional spatial filter dependent on a CT number represented by a focused pixel. The CT number is an example of a value representing a property. Any other value representing a property, for example, a standard deviation of a CT number, a first derivative thereof, a second derivative thereof, or a time difference thereof may be used to realize a four-dimensional spatial filer dependent on the CT number.

Fig. 23 describes an example of four-dimensional spatial filtering dependent on the property of a neighborhood. A CT number is used as an example of a value representing a property. Any other value representing a property, for example, a standard deviation of a CT number, a first derivative thereof, a second derivative thereof, or a time difference thereof may be used to realize four-dimensional spatial filtering dependent on the property of a neighborhood.

## Claims

**1.** An image processing apparatus (1) comprising:

an image input device for receiving a time-varying three-dimensional image;
a spatial filter device (3) for performing four-dimensional spatial filtering in the direction of a time axis and the directions of spatial axes; and

an image output/display device (6) for transmitting or displaying a spatially filtered three-dimensional image.

2. An image processing apparatus (1) comprising:

an image input device for receiving a time-varying N-1-dimensional image defined using N-1 time-varying independent parameters as a base;
a spatial filter device (3) for performing N-dimensional spatial filtering in the direction of a time axis and the directions of spatial axes; and
an image output/display device (6) for transmitting or displaying a spatially filtered N-1-dimensional image.

3. An image processing apparatus (1) comprising:

an image input device for receiving a time-varying three-dimensional image;
a spatial filter device (3) for selecting pixels that mutually neighbor in the direction of a time axis and the directions of spatial axes, and performing adaptive four-dimensional spatial filtering on the selected neighboring pixels; and
an image output/display device (6) for transmitting or displaying a spatially filtered three-dimensional image.

4. An image processing apparatus (1) comprising:

an image input device for receiving a time-varying N-1-dimensional image defined using N-1 time-varying independent parameters as a base;
a spatial filter device (3) for selecting pixels that mutually neighbor in the direction of a time axis and the directions of spatial axes, and performing adaptive N-dimensional spatial filtering on the selected neighboring pixels; and
an image output/display device (6) for transmitting or displaying a spatially filtered N-1-dimensional image.

5. The image processing apparatus (1) according to any of Claims 1 to 4, comprising the spatial filter device (3) which selects pixels whose values are statistically close to the value of a focused image to be aligned with the center of a spatial filter as the selected neighboring pixels.

6. An X-ray CT apparatus (100) comprising:

a data acquisition device (20) for rotating an X-ray generator (21) and a two-dimensional X-ray area detector (24), which has a matrix structure and is opposed to the X-ray generator (21), about a center of rotation located between the X-ray generator (21) and two-dimensional X-ray area detector (24) so as to acquire projection data items of a subject lying down between the X-ray generator (21) and two-dimensional X-ray area detector (24);
an image reconstruction device (3) for reconstructing an image according to the acquired projection data items;
a post-processing device (3) for performing post-processing on a reconstructed tomographic image;
a tomographic image display device (6) for displaying the tomographic image having undergone the post-processing; and
a radiographic condition designation device (2) for designating radiographic conditions, wherein:

the post-processing device (3) spatially filters a time-varying three-dimensional image, which is produced through tomography, in x, y, and z directions where the z direction is a direction perpendicular to an xy plane that is a plane on which a data acquisition system rotates or a plane expressed by a tomographic image.

7. An X-ray CT apparatus (100) comprising:

a data acquisition device (20) for rotating an X-ray generator (21) and a two-dimensional X-ray area detector (24), which has a matrix structure and is opposed to the X-ray generator (21), about a center of rotation located between the X-ray generator (21) and two-dimensional X-ray area detector (24) so as to acquire projection data items of a subject lying down between the X-ray generator (21) and two-dimensional X-ray area detector (24);
an image reconstruction device (3) for reconstructing an image according to the acquired projection data items;
a post-processing device (3) for performing post-processing on a reconstructed tomographic image;
a tomographic image display device (6) for displaying the tomographic image having undergone the post-processing; and
a radiographic condition designation device (2) for designating radiographic conditions, wherein:

the X-ray CT apparatus (100) further comprises a preprocessing device for spatially filtering time-varying projection data items, which are produced through tomography, in the direction of a time axis and the directions of spatial axes, that is, the direction of channels, the direction of detector arrays, and a direction determined by a view angle.

8. An X-ray CT apparatus (100) comprising:

a data acquisition device (20) for rotating an X-ray generator (21) and a two-dimensional X-ray area detector (24), which has a matrix structure and is opposed to the X-ray generator (21), about a center of rotation located between the X-ray generator (21) and two-dimensional X-ray area detector (24) so as to acquire projection data items of a subject lying down between the X-ray generator (21) and two-dimensional X-ray area detector (24); an image reconstruction device (3) for reconstructing an image according to the acquired projection data items; a post-processing device (3) for performing post-processing on a reconstructed tomographic image; a tomographic image display device (6) for displaying the tomographic image having undergone the post-processing; and a radiographic condition designation device (2) for designating radiographic conditions, wherein:

the post-processing device (3) includes: a device for selecting pixels, which mutually neighbor in the direction of a time axis and the directions of spatial axes, that is, in x, y, and z directions where the z direction is a direction perpendicular to an xy plane that is a plane on which a data acquisition system rotates or a plane expressed by a tomographic image, from pixels constituting time-varying three-dimensional image data produced through tomography; and a device for performing adaptive spatial filtering on the selected neighboring pixels.

9. An X-ray CT apparatus (100) comprising:

a data acquisition device (20) for rotating an X-ray generator (21) and a two-dimensional X-ray area detector (24), which has a matrix structure and is opposed to the X-ray generator (21), about a center of rotation located between the X-ray generator (21) and two-dimensional X-ray area detector (24) so as to acquire projection data items of a subject lying down between the X-ray generator (21) and two-dimensional X-ray area detector (24); an image reconstruction device (3) for reconstructing an image according to the acquired projection data items; a post-processing device (3) for performing post-processing on a reconstructed tomographic image; a tomographic image display device (6) for displaying the tomographic image having undergone the post-processing; and a radiographic condition designation device (2) for designating radiographic conditions, wherein:

the X-ray CT apparatus (100) further comprises a preprocessing device including: a device for selecting pixels, which mutually neighbor in the direction of a time axis and the directions of spatial axes, that is, in x, y, and z directions where the z direction is a direction perpendicular to an xy plane that is a plane on which a data acquisition system rotates or a plane expressed by a tomographic image, from pixels constituting time-varying projection data items produced through tomography; and a device for performing adaptive spatial filtering on the selected neighboring pixels.

10. The X-ray CT apparatus (100) according to any of Claims 6 to 9, comprising the post-processing device (3) which selects pixels whose values are statistically close to the value of a focused pixel to be aligned with the center of a spatial filter as the selected neighboring pixels.

# FIG. 1

100

y
↑
→ x
⊗
z

1

6 MONITOR

2 INPUT

3 CPU

5 DATA COLLECTION BUFFER

7 STORAGE

20

15 X-RAY TUBE

26 ROTATOR CONTROLLER

21 COLLIMATOR

23

22 X-RAY CONTROLLER

24

X-RAY DETECTOR

DAS 25

30

CONTROL UNIT

29

SCANNER GANTRY TILT CONTROLLER 27

12

10

FIG. 2

## FIG. 3

Start

PERFORM DATA ACQUISITION — S1

PERFORM PREPROCESSING — S2

PERFORM BEAM HARDENING COMPENSATION — S3

PERFORM Z FILTER CONVOLUTION — S4

PERFORM RECONSTRUCTION FUNCTION CONVOLUTION — S5

PERFORM THREE-DIMENSIONAL BACK PROJECTION — S6

PERFORM POST-PROCESSING — S7

PERFORM FOUR-DIMENSIONAL SPATIAL FILTERING — S8

PERFORM TOMOGRAPHIC IMAGE DISPLAY, THREE-DIMENSIONAL IMAGE DISPLAY, AND THREE-DIMENSIONAL IMAGE MEASUREMENT — S9

end

# FIG. 4

```
        ┌──────────────────┐
        │      Start       │
        └──────────────────┘
                 │
                 ▼                      Step  S21
        ┌──────────────────┐
        │PERFORM OFFSET NULLING│
        └──────────────────┘
                 │
                 ▼                      Step  S22
        ┌──────────────────┐
        │ PERFORM LOGARITHMIC│
        │    CONVERSION     │
        └──────────────────┘
                 │
                 ▼                      Step  S23
        ┌──────────────────┐
        │   PERFORM X-RAY   │
        │  DOSE CORRECTION  │
        └──────────────────┘
                 │
                 ▼                      Step  S24
        ┌──────────────────┐
        │PERFORM SENSITIVITY│
        │    CORRECTION     │
        └──────────────────┘
                 │
                 ▼
        ┌──────────────────┐
        │       end        │
        └──────────────────┘
```

# FIG. 5

STEP S6

Start three-dimensional
back projection

S61

SAMPLE PROJECTION DATA ITEMS Dr REPRESENTING
PIXEL POINTS IN FIELD OF VIEW P

S62

MULTIPLY PROJECTION DATA ITEMS Dr BY EITHER OF
CONE-BEAM RECONSTRUCTION WEIGHTING COEFFICIENTS SO
AS TO PRODUCE BACK PROJECTION DATA ITEMS D2

S63

ADD BACK PROJECTION DATA ITEMS D2 TO BACK
PROJECTION DATA ITEMS D3 PIXEL BY PIXEL

S64

HAVE BACK PROJECTION DATA ITEMS
D2 INCLUDED IN ALL VIEWS, WHICH ARE
REQUIRED FOR RECONSTRUCTION OF
TOMOGRAPHIC IMAGE, BEEN ADDED?

NO

YES

end

# FIG. 6

(a)

origin (0, 0)

L0
L63
L127
L191
L255
L319
L383
L447
L511

(511, 511)

P

IC

(b)

Xy plane

axis of rotation
IC

z axis

L0
L63
L127
L191
L255
L319
L383
L447
L511

P

24

## FIG. 7

J/2

24

direction
of detector
arrays

T127
T63
T0

T511
T447
T382
T319
T255
T191

direction of channels

## FIG. 8

P    view= 0°

Dr(0° ,x,y)

FIG. 9

P   view＝0°

D2(0°,x,y)

FIG.10

D3(x,y)   D2 (0°,x,y)   D2 (0.36°,x,y)   D2 (view,x,y)

## FIG.11

(a)

L0
L63
L127
L191
L255
L319
L383
L447
L511

P

IC

21

y
z → x

(b)

21 X 線管

P

L0
L63
L127
L191
L255

L319
L383
L447
L511

Xy plane

axis of rotation
IC

z axis

24

y
x → z

## FIG.12

(a)

tomographic image

(b)

three-dimensional image

three-dimensional filter

FIG.13

(a)

x, y

tomographic images $c(t1, z1), c(t1, z2),$ etc., and $c(t1, zN)$ reconstructed at time instant $t1$

tomographic images $c(t2, z1), c(t2, z2),$ etc., and $c(t2, zN)$ reconstructed at time instant $t2$

tomographic images $c(t3, z1), c(t3, z2),$ etc., and $c(t3, zN)$ reconstructed at time instant $t3$

z

(b)

x, y

tomographic images $h(t1, z1), h(t1, z2),$ and $h(t1, z3)$ reconstructed at time instant $t1$

tomographic images $h(t2, z1), h(t2, z2),$ and $h(t2, z3)$ reconstructed at time instant $t2$

tomographic images $h(t3, z1), h(t3, z2),$ and $h(t3, z3)$ reconstructed at time instant $t3$

z

# FIG.14

(a)

(b)

FIG.15

tn−1          tn          tn+1

→ Time

0 → x
y   z

three-dimensional image expressing state of subject attained at time instant tn−1

three-dimensional image expressing state of subject attained at time instant tn

three dimensional image expressing state of subject attained at time instant tn+1

*1   *2   *3

0−1−1
0−1−2
0−1−3

1−1−1
1−1−2
1−1−3

*1 swept position on t axis

*2 swept position on z axis

*3 swept position on y axis

0−2−1
0−2−2
0−2−3

1−2−1
1−2−2
1−2−3

33

## FIG.16

(a)

tn− 1    tn    tn+ 1

three-dimensional spatial
filter employed at time
instant tn-1

three-dimensional spatial
filter employed at time
instant tn

three-dimensional spatial
filter employed at time
instant tn+1

(b)

$t_{n-1}$    $t_n$    $t_{n+1}$

z 上

z 中

z 下

$3 \times 3 \times 3 \times 3 - 1 = 80$ pixels

▨ : neighbouring pixel

■ : focused pixel

## FIG.17

tn− 2    tn− 1    tn    tn+ 1    tn+ 2    time

(a)

three-dimensional spatial filter employed at time instant $t_{n-2}$

three-dimensional spatial filter employed at time instant $t_{n-1}$

three-dimensional spatial filter employed at time instant $t_n$

three-dimensional spatial filter employed at time instant $t_{n+1}$

three-dimensional spatial filter employed at time instant $t_{n+2}$

tn− 2    tn− 1    tn    tn+ 1    tn+ 2    time

(b)

z 上

z 中

z 下

$5 \times 5 \times 5 \times 5 - 1 = 624$ pixels

☒ : neighbouring pixel

■ : focused pixel

# FIG.18

$3 \times 3 \times 3 \times 3 - 1 = 80$ pixels

a = 0.36

b = 0.05    b × (1 + 6 + 1) = 0.40

c = 0.01    c × (6 + 12 + 6) = 0.24    } Total 1.00

## FIG.19

c:1

b:1
c:6

a:1
b:6
c18

b:1
c:6

c:1

total
a:1
b:8
c:32

a=0.76       a=0.76
b=0.01       b×(1+6+1)=0.08
c=0.005      c×(1+6+18+6+1)=      total 1.00

# FIG.20

(a)

time

tn−1  tn  tn+1  tn+2

0 → x
y  z

z 上
z 中
z 下

c:1

b:1
c:6

a:1
b:6
c18

b:1
c:6

c:1

coefficients
defined by first filter

coefficients
defined by second filter

Total

a:1
b:8
c:32

a=0.28
b=0.05  b×8=0.4  } Total
c=0.01  c×32=0.32  } 1.00

a=1
b=0
c=0

(b)

weighting coefficient

second filter    first filter    second filter

1

0

Th1          Th2              Th3      Th4
threshold    threshold        threshold threshold
1            2                3        4

CT number

# FIG.21

(a)

c:1

b:1
c:6

a:1
b:6
c18

b:1
c:6

c:1

coefficients defined by
contrast enhancement first filter

a = 2.12
b = − 0.1
c = − 0.01

a = 2.12
b × 8 = − 0.8 } total 1.00
c × 32 = − 0.32

coefficients
defined by second filter

a = 0.76
b = 0.01
c = 0.005

a = 0.76
b = (1 + 6 + 1) = 0.08 } total 1.00
c = (1 + 6 + 18 + 6 + 1) = 0.16

(b)

weighting coefficient

first filter    Second filter    first filter

1

0

Th1
threshold 1

Th2
threshold 2

Th3
threshold 3

Th4
threshold 4

CT number

# FIG.22

```
              ( Start )
                   │
                   ▼
┌─────────────────────────────────────────┐   ── Step F1
│ SELECT SPATIAL FILTERS Fk, WHICH ARE APPLIED TO │
│ RANGES R1, R2, ETC., AND TN OF PIXEL VALUES     │
│ (CT NUMBERS) TO BE SPATIALLY FILTERED, AMONG    │
│ MANY SPATIAL FILTERS                            │
└─────────────────────────────────────────┘
                   │                        ── Step F2
                   ▼
        ┌──────────────────────────┐
        │ INITIALIZE i, j, AND k TO 1s │
        └──────────────────────────┘
                   │
                   ▼
┌────────────────────────────────────────┐  ── Step F3
│      DOES CT NUMBER OF FOCUSED PIXEL,   │                ── Step F4
│  TO WHICH SPATIAL FILTER IS CONVOLUTED, │  NO  ┌──────────────────┐
│   FALL WITHIN RANGE Rk OF PIXEL         │ ───► │ INCREMENT k BY 1  │
│   VALUES (CT NUMBERS)? (IMAGE DATA      │      └──────────────────┘
│   (TOMOGRAPHIC IMAGE DATA) G(i,j) HAS   │
│    N PIXELS LINED IN ROWS AND COLUMNS)  │
└────────────────────────────────────────┘
                   │ YES                       ── Step F5
                   ▼
┌──────────────────────────────────────────┐
│ CONVOLUTE SPATIAL FILTER Fk ASSOCIATED WITH │
│  RANGE Rk OF PIXEL VALUES (CT NUMBERS)      │
└──────────────────────────────────────────┘
  ── Step F7          │              ── Step F6
┌──────────────┐ NO   ▼
│ INCREMENT i BY 1 │◄─ ◇ IS i EQUAL TO N? ◇
└──────────────┘      │ YES             ── Step F8
                      ▼
            ┌──────────────────┐
            │ INITIALIZE i TO 1 │
            └──────────────────┘
  ── Step F10         │              ── Step F9
┌──────────────┐ NO   ▼
│ INCREMENT j BY 1 │◄─ ◇ IS j EQUAL TO N? ◇
└──────────────┘      │ YES
                      ▼
                   ( end )
```

# FIG.23

```
                    ( start )
                        │
                        ▼                          ──── Step F101
┌──────────────────────────────────────────────────────────────┐
│   DETERMINE THRESHOLD a TO BE USED TO DISCRIMINATE VALUE       │
│   OF FOCUSED PIXEL FROM PIXEL VALUES IN NEIGHBORHOOD AND       │
│   PARAMETERS TO BE USED TO INDICATE SIZE bxc OF NEIGHBORHOOD   │
└──────────────────────────────────────────────────────────────┘
                        │
                        ▼              ──── Step F102
            ┌──────────────────────────────┐
            │   INITIALIZE i AND j TO 1S    │
            └──────────────────────────────┘
                        │
                        ▼                          ──── Step F103
┌──────────────────────────────────────────────────────────────┐
│   CALCULATE MEANS m OF PIXEL VALUES IN EACH OF NEIGHBORHOODS   │
│   (G(i-b,j)),G(i+b,j)) AND (G(i,j-c)),G(i,j+c)) OF FOCUSED     │
│   PIXEL G(i,j), AND STANDARD DEVIATION s OF EACH OF PIXEL      │
│              VALUES IN EACH OF NEIGHBORHOODS.                  │
└──────────────────────────────────────────────────────────────┘
                        │                ──── Step F104
                        ▼
              ╱ DOES VALUE OF FOCUSED ╲
             ╱   PIXEL G(i,j) FALL WITHIN ╲
            ⟨ RANGE OF PIXEL VALUES (CT NUMBERS) ⟩──── NO
             ╲  FROM (m-a·s) TO (m+a·s)? ╱
                        │
              YES   ──── Step F105              ──── Step F106
                        ▼                              ▼
            ┌──────────────────────┐      ┌──────────────────────┐
            │  CONVOLUTE FILTER F1  │      │  CONVOLUTE FILTER F2  │
            └──────────────────────┘      └──────────────────────┘
                        │                              │
                        ▼◄─────────────────────────────┘
    ──── Step F108           ──── Step F107
┌──────────────────┐   NO   ╱                ╲
│ INCREMENT i BY 1 │◄──────⟨  IS i EQUAL TO N? ⟩
└──────────────────┘        ╲                ╱
        ▲                        │
        │                 YES  ──── Step F109
        │                        ▼
        │              ┌──────────────────────┐
        │              │   INITIALIZE i TO 1   │
        │              └──────────────────────┘
        │                        │
    ──── Step F111                ▼  ──── Step F110
┌──────────────────┐   NO   ╱                ╲
│ INCREMENT j BY 1 │◄──────⟨  IS j EQUAL TO N? ⟩
└──────────────────┘        ╲                ╱
                                 │
                               YES
                                 ▼
                             ( end )
```

# FIG.24

Tomographic image

three-dimensional mpr display

lung field

z direction

heart

lung field

scout image

blood vessels in lung fields

heart

lung field

three-dimensional display

# FIG.25

(a)

$m-a\cdot s$     $m+a\cdot s$

frequency

distribution of pixel values
(ct numbers) in neighborhood

0

m

pixel value (ct number) or
value indicating property

value of focused pixel

(b)

$m-a\cdot s$     $m+a\cdot s$

frequency

distribution of pixel values
(ct numbers) in neighborhood

0

m

pixel value (ct number) or
value indicating property

value of focused pixel

(c)

b     b

c

c

focused pixel

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Oplus E. New Technological Communications Inc, November 1988, 144-145